(19) Europäisches Patentamt
European Patent Office
Office européen des brevets

(11) EP 2 832 730 A1

(12) EUROPEAN PATENT APPLICATION
published in accordance with Art. 153(4) EPC

(43) Date of publication:
04.02.2015 Bulletin 2015/06

(21) Application number: 13770182.7

(22) Date of filing: 29.03.2013

(51) Int Cl.:
*C07D 277/20* (2006.01) *A61K 31/426* (2006.01)
*A61K 47/32* (2006.01) *A61K 47/38* (2006.01)
*A61P 13/10* (2006.01)

(86) International application number:
PCT/JP2013/059486

(87) International publication number:
WO 2013/147134 (03.10.2013 Gazette 2013/40)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: 30.03.2012 US 201261618076 P
15.03.2013 JP 2013053462

(71) Applicant: **Astellas Pharma Inc.**
**Chuo-ku**
**Tokyo 103-8411 (JP)**

(72) Inventors:
- **KASASHIMA, Yuki**
  **Tokyo 103-8411 (JP)**
- **YOSHIHARA, Keiichi**
  **Tokyo 103-8411 (JP)**
- **ITO, Yoshitaka**
  **Tokyo 103-8411 (JP)**
- **YOSHIDA, Takatsune**
  **Tokyo 103-8411 (JP)**
- **MATSUI, Yumi**
  **Tokyo 103-8411 (JP)**

(74) Representative: **Hoffmann Eitle**
**Patent- und Rechtsanwälte PartmbB**
**Arabellastraße 30**
**81925 München (DE)**

# (54) MIRABEGRON-CONTAINING PHARMACEUTICAL COMPOSITION

(57) Provided is a mirabegron-containing pharmaceutical composition in which the leakage of mirabegron can be inhibited when the pharmaceutical composition is dispersed in a liquid, and in which the change in pharmacokinetics caused by the presence or absence of food intake is decreased. The pharmaceutical composition comprises an acid addition salt of alkyl sulfuric acid and mirabegron, and a base for modified release.

Figure 1

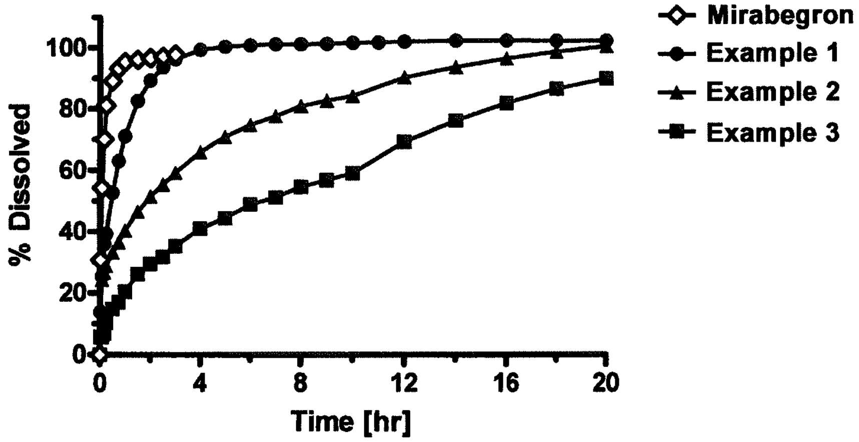


EP 2 832 730 A1

Printed by Jouve, 75001 PARIS (FR)

## Description

TECHNICAL FIELD

The present invention relates to a pharmaceutical composition comprising an acid addition salt of alkyl sulfuric acid and mirabegron, and a base for modified release. Further, the present invention relates to a pharmaceutical composition comprising an acid addition salt of alkyl sulfuric acid and mirabegron. Furthermore, the present invention relates to an acid addition salt of alkyl sulfuric acid and mirabegron.

BACKGROUND ART

Mirabegron is a compound that is also known as YM178, and its chemical name is (R)-2-(2-aminothiazol-4-yl)-4'-{2-[(2-hydroxy-2-phenylethyl)amino]ethyl}acetanilide, with the following chemical structure. Mirabegron or its salts have a $\beta_3$-adrenergic receptor agonist activity, and are known to be useful as a therapeutic agent for overactive bladder (Patent literatures 1 to 3).

[Chem. 1]

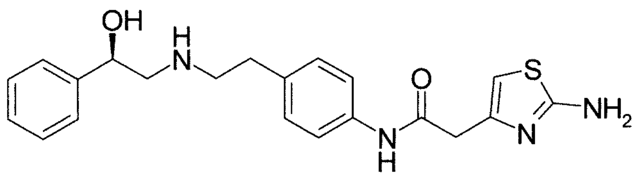

Mirabegron has been launched on the market in Japan and the United States as a therapeutic agent for overactive bladder, and is sold as "Betanis (registered trademark) tablet" in Japan and as "MYRBETRIQ (registered trademark) tablet" in the United States. Patent literature 2 discloses mirabegron dihydrochloride as an acid addition salt of mirabegron.

In clinical studies carried out during the development phase of mirabegron, it is known that the pharmacokinetics of mirabegron varies according to the presence or absence of food intake (Patent literature 4). The change in pharmacokinetics caused by the presence or absence of food intake naturally affects its function and effects. Particularly, in medicaments, because there is a possibility that function and effects different from predicted ones may result in unpredictable adverse effects, it is necessary to predict certain function and effects. Therefore, it has been strongly desired to develop medicaments in which the change in pharmacokinetics caused by the presence or absence of food intake is minimized, and it is known that the change in pharmacokinetics of mirabegron caused by the presence or absence of food intake can be reduced by a controlled release of drug using various additives (Patent literature 4). However, as the Medical Package Insert of Betanis (registered trademark) tablet with the modified release of drug states that "it is orally administered once daily after a meal", the Betanis (registered trademark) tablet currently provided in the medical field is a formulation in which the usage is limited (Non-patent literature 1).

From another perspective, the pharmaceutical formulation of mirabegron which is currently placed on the market is tablets, and it is desired to develop various dosage forms such as liquids, suspensions, emulsions, powders, and granules, form the viewpoint of improving the drug dosing compliance for children. Further, in a guideline (Non-patent literature 3) by The International Conference on Harmonisation of Technical Requirements for Registration of Pharmaceuticals for Human Use (ICH), a child-related guidance (Non-patent literature 4) by the U.S. Food and Drug Administration (FDA), and the Pediatric Research Equity Act (PREA)(Non-patent literature 5) by the FDA, pediatric dosage forms, such as liquids, suspensions, or emulsions, which are easy to take and capable of controlling a dose are desired.

However, mirabegron has extremely strong bitterness, and it is necessary to inhibit the bitterness to provide pediatric dosage forms such as liquids.

Therefore, to provide a formulation capable of further reducing the change in pharmacokinetics caused by the presence or absence of food intake, without the limitation of the usage, a further technical development has been desired.

Further, to provide a formulation in which the bitterness is inhibited or reduced by controlling the dissolution of the drug, even when mirabegron is dispersed in a solvent, or is stored as liquids, suspensions, or emulsions while inhibiting the bitterness of mirabegron, a further technical development has been desired.

Furthermore, a technical development for inhibiting the dissolution or leakage of the drug before administration, caused by its storage as liquids, suspensions, or emulsions, is desired.

It is known that a pharmaceutical composition which is capable of maintaining a state of solution and can

achieve a modified release can be provided by adding, to an ionic pharmaceutically active substance, an equimolar or more of an ionic compound which has the opposite charge and can increase the hydrophobicity of the active substance, and that alkyl sulfuric acid, such as sodium lauryl sulfate or sodium myristyl sulfate, may be used as the ionic compound (Patent literatures 5 and 6). However, these references do not concretely disclose the applicability to mirabegron or its salts.

CITATION LIST

PATENT LITERATURE

[Patent literature 1] WO 2004/041276
[Patent literature 2] WO 99/20607
[Patent literature 3] WO 03/037881
[Patent literature 4] WO 2010/038690
[Patent literature 5] WO 99/33489
[Patent literature 6] WO 99/33491

NON-PATENT LITERATURE

[Non-patent literature 1] "Betanis Tablet" Package Insert
[Non-patent literature 2] "Betanis Tablet" Interview Form
[Non-patent literature 3] ICH Guidance: ICH, Guidance for Industry: E11 Clinical Investigation of Medical Products in the Pediatric Population (Dec. 2000)
[Non-patent literature 4] FDA: Guidance for Industry: General Consideration for Pediatric Pharmacokinetic Studies for Drugs and Biological Products (Nov. 1998)
[Non-patent literature 5] PREA: Guidance for Industry: How to Comply with the Pediatric Research Equity Act (Sep. 2005)

SUMMARY OF INVENTION

TECHNICAL PROBLEM

An object of the present invention is to provide a pharmaceutical composition comprising mirabegron or its salts with inhibited or reduced bitterness, by reducing or inhibiting the dissolution or leakage of mirabegron, even when the pharmaceutical composition is stored as liquids, suspensions, or emulsions, or even when mirabegron is suspended in a solvent, or is stored. Another object of the present invention is to provide a pharmaceutical composition in which the change in pharmacokinetics caused by the presence or absence of food intake is reduced. In another embodiment, an object of the present invention is to provide a pharmaceutical composition comprising an acid addition salt of alkyl sulfuric acid and mirabegron. In still another embodiment, an object of the present invention is to provide a pharmaceutical composition comprising an acid addition salt of alkyl sulfuric acid and mirabegron, which is useful as an active ingredient for the treatment of overactive bladder. In still another embodiment, an object of the present invention is to provide an acid addition salt of alkyl sulfuric acid and mirabegron, in which the change in pharmacokinetics caused by the presence or absence of food intake is reduced.

SOLUTION TO PROBLEM

Under those circumstances, the present inventors conducted studies by focusing that when a pharmaceutical composition comprising mirabegron was stored as liquids, suspensions, or emulsions, the dissolution or leakage of mirabegron was reduced or inhibited, and that when the pharmaceutical composition was suspended in a solvent and stored, the dissolution or leakage of mirabegron was reduced or inhibited. As a result, the present inventors focused that a pharmaceutical composition comprising an acid addition salt of alkyl sulfuric acid and mirabegron, including mirabegron didodecyl sulfate, mirabegron ditetradecyl sulfate, mirabegron dihexadecyl sulfate, and mirabegron monododecyl sulfate, as a form of mirabegron with decreased solubility, exhibited the desired effects of the present invention in vitro and in vivo tests; and that the change in pharmacokinetics of mirabegron caused by the presence or absence of

food intake could be reduced by modified release of drug; and found that the pharmaceutical composition comprising an acid addition salt of alkyl sulfuric acid and mirabegron, such as mirabegron didodecyl sulfate, mirabegron ditetradecyl sulfate, mirabegron dihexadecyl sulfate, and mirabegron monododecyl sulfate, as a form of mirabegron with decreased solubility, exhibited the desired effects of the present invention in vitro and in vivo tests; and completed the present invention.

The present invention relates to the following:

[1] A pharmaceutical composition comprising an acid addition salt of alkyl sulfuric acid and mirabegron, and a base for modified release.
[2] The pharmaceutical composition of [1], wherein the alkyl sulfuric acid is an acid selected from the group consisting of dodecyl sulfuric acid, tetradecyl sulfuric acid, and hexadecyl sulfuric acid.
[3] The pharmaceutical composition of [2], wherein the acid addition salt of alkyl sulfuric acid and mirabegron is mirabegron dodecyl sulfate.
[4] The pharmaceutical composition of any one of [1] to [3], wherein a molar ratio of mirabegron to alkyl sulfuric acid is 1:1 to 1:2.
[5] The pharmaceutical composition of [1], wherein the base for modified release is a water-soluble polymer or a water-insoluble substance.
[6] The pharmaceutical composition of [5], wherein the base for modified release is a water-insoluble substance.
[7] The pharmaceutical composition of [1] or [6], wherein the base for modified release is a water-insoluble cellulose ether and/or a water-insoluble acrylate copolymer.
[8] The pharmaceutical composition according to [7], wherein the water-insoluble cellulose ether is ethyl cellulose.
[9] The pharmaceutical composition of [7], wherein the water-insoluble acrylate copolymer is one substance or two or more substances selected from an ethyl acrylate/methyl methacrylate/trimethylammoniumethyl methacrylate chloride copolymer and an ethyl acrylate/methyl methacrylate copolymer.
[10] The pharmaceutical composition according to any one of [5] to [9], wherein a content of the water-insoluble substance is 0.1 W/W% or more and 1000 W/W% or less, with respect to the weight of the acid addition salt of alkyl sulfuric acid and mirabegron.
[11] The pharmaceutical composition of any one of [1] to [10], wherein a dissolution rate of mirabegron after 30 minutes from the beginning of a dissolution test is approximately less than 85%.
[12] The pharmaceutical composition of [11], wherein a dissolution rate of mirabegron after 1.5 hours from the beginning of a dissolution test is approximately 70% or less.
[13] The pharmaceutical composition of any one of [1] to [12], wherein a rate of decrease of a maximum blood drug concentration (Cmax) when administered after eating a meal, in comparison with a Cmax when administered in a fasted state, is approximately 30% or less.
[14] The pharmaceutical composition of any one of [1] to [13], wherein a rate of decrease of an area under a blood drug concentration versus time curve (AUC) when administered after eating a meal, in comparison with an AUC when administered in a fasted state, is approximately 30% or less.
[15] The pharmaceutical composition of any one of [1] to [14], wherein its dosage form is selected from the group consisting of granules, powders, liquids, suspensions, and emulsions.
[16] The pharmaceutical composition of [15], wherein the dosage form is selected from liquids, suspensions, and emulsions.
[17] The pharmaceutical composition of any one of [1] to [16], which is a therapeutic agent for overactive bladder.
[18] A process of manufacturing a pharmaceutical composition, comprising adding a base for modified release to an acid addition salt of alkyl sulfuric acid and mirabegron.
[19] A process of manufacturing a pharmaceutical composition, comprising the steps of:

(1) dissolving mirabegron in a solvent,
(2) adding alkyl sulfuric acid to the resulting mixture prepared in (1), and
(3) adding a base for modified release to the resulting mixture prepared in (2).

[20] An acid addition salt of alkyl sulfuric acid and mirabegron.
[21] The acid addition salt of [20], wherein the alkyl sulfuric acid is an acid selected from the group consisting of dodecyl sulfuric acid, tetradecyl sulfuric acid, and hexadecyl sulfuric acid.
[22] The acid addition salt of [21], wherein the acid addition salt of alkyl sulfuric acid and mirabegron is mirabegron dodecyl sulfate.
[23] The acid addition salt of any one of [20] to [22], wherein a molar ratio of mirabegron to alkyl sulfuric acid is 1:1 to 1:2.
[24] Use of the acid addition salt of any one of [20] to [23] for the treatment of overactive bladder.
[25] A method for treating overactive bladder, comprising administering to a subject in need thereof the acid addition

salt of any one of [20] to [23] in an amount effective therefor.
[26] Use of the acid addition salt of any one of [20] to [23] for the inhibition of leakage of mirabegron during the storage of mirabegron dispersed in water or a xanthan gum solution.
[27] Use of the acid addition salt of any one of [20] to [23] for the manufacture of a pharmaceutical composition in which the change in pharmacokinetics is reduced regardless of the presence or absence of food intake.
[28] Use of the acid addition salt of any one of [20] to [23] for the inhibition of bitterness.
[29] Use of the acid addition salt of any one of [20] to [23] for the manufacture of a pharmaceutical composition for the treatment of overactive bladder.

## ADVANTAGEOUS EFFECTS OF INVENTION

The pharmaceutical composition of the present invention comprises an acid addition salt of alkyl sulfuric acid and mirabegron, and a base for modified release. The pharmaceutical composition of the present invention can provide a pharmaceutical composition in which, in comparison with mirabegron, its solubility or dissolution rate is decreased or reduced, and in which the dissolution or leakage of mirabegron can be reduced or inhibited when the pharmaceutical composition is suspended or dispersed in a solvent and stored. Further, the present invention can provide a mirabegron-containing pharmaceutical composition in which the change in pharmacokinetics caused by the presence or absence of food intake is decreased, in comparison with mirabegron. Furthermore, the present invention can inhibit or reduce the bitterness, in comparison with mirabegron, when it is used as orally disintegrating tablets, granules, powders, liquids, suspensions, and emulsions.
The acid addition salt of alkyl sulfuric acid and mirabegron of the present invention can provide a pharmaceutical composition in which, in comparison with mirabegron, its solubility or dissolution rate is decreased or reduced, and in which the dissolution or leakage of mirabegron can be reduced or inhibited when the pharmaceutical composition is suspended in a solvent and stored. Further, the present invention can provide a mirabegron-containing pharmaceutical composition in which the change in pharmacokinetics caused by the presence or absence of food intake is small, in comparison with mirabegron. Furthermore, the present invention can inhibit or reduce the bitterness, in comparison with mirabegron, when it is used as orally disintegrating tablets, granules, powders, liquids, suspensions, and emulsions.

## BRIEF DESCRIPTION OF THE DRAWINGS

Figure 1 is a graph showing the results of a dissolution test of the acid addition salts of the present invention prepared in Examples 1 to 3 (control: mirabegron), carried out in Experimental Example 2.
Figure 2 is a graph showing the results of a dissolution test of the acid addition salts of the present invention prepared in Examples 4 and 5 (Comparative Examples 2 to 5 for comparison), carried out in Experimental Example 3.
Figure 3 is a graph showing time courses of plasma concentrations of mirabegron in fasted and fed states, in a pharmacokinetics test (dogs) of the acid addition salt of the present invention prepared in Example 1, carried out in Experimental Example 4.
Figure 4 is a graph showing time courses of plasma concentrations of mirabegron in fasted and fed states, in a pharmacokinetics test (dogs) of the drug aqueous solution prepared in Comparative Example 1, carried out in Experimental Example 4.
Figure 5 is a graph showing the results of a dissolution test of a suspension prepared by dispersing a liquid of the acid addition salt of the present invention prepared in Example 1 to a xanthan gum solution, after being stored at a cool place (5°C) or at room temperature for a month (control: suspension before the storage), in a stability test of the liquid of the acid addition salt of the present invention prepared in Example 1, carried out in Experimental Example 5.
Figure 6 is a graph showing the results of a dissolution test of the pharmaceutical compositions of the present invention prepared in Examples 7 to 11, carried out in Experimental Example 6.
Figure 7 is a graph showing the results of a dissolution test of the pharmaceutical compositions of the present invention prepared in Examples 12 to 15, carried out in Experimental Example 6.
Figure 8 is a graph showing time courses of plasma concentrations of mirabegron in fasted and fed states, in a pharmacokinetics test (dogs) of the acid addition salt of the present invention prepared in Example 7, carried out in Experimental Example 7.
Figure 9 is a graph showing time courses of plasma concentrations of mirabegron in fasted and fed states, in a pharmacokinetics test (dogs) of the acid addition salt of the present invention prepared in Example 15, carried out in Experimental Example 7.
Figure 10 is a graph showing the results of a dissolution test of a suspension prepared by dispersing a liquid of the

acid addition salt of the present invention prepared in Example 7 to a xanthan gum solution, after being stored at a cool place (5°C) or at room temperature for a month (control: suspension before the storage), in a stability test of the liquid of the acid addition salt of the present invention prepared in Example 7, carried out in Experimental Example 8.

Figure 11 is a graph showing the results of a dissolution test of a suspension prepared by dispersing a liquid of the acid addition salt of the present invention prepared in Example 15 to water, after being stored at room temperature for 2 weeks (control: suspension before the storage), in a stability test of the liquid of the acid addition salt of the present invention prepared in Example 15, carried out in Experimental Example 8.

Figure 12 is a graph showing the results of a dissolution test of a suspension prepared by dispersing a liquid of the pharmaceutical composition prepared in Comparative Example 6 to water, after being stored at room temperature for 2 weeks (control: suspension before the storage), in a stability test of the liquid of the pharmaceutical composition prepared in Comparative Example 6, carried out in Experimental Example 8.

Figure 13 is a graph showing the results of a dissolution test of a suspension prepared by dispersing a liquid of the pharmaceutical composition prepared in Comparative Example 7 to water, after being stored at room temperature for 2 weeks (control: suspension before the storage), in a stability test of the liquid of the pharmaceutical composition prepared in Comparative Example 7, carried out in Experimental Example 8.

Figure 14 is a graph showing the results of a dissolution test of a suspension prepared by dispersing a liquid of the pharmaceutical composition prepared in Comparative Example 8 to water, after being stored at room temperature for 2 weeks (control: suspension before the storage), in a stability test of the liquid of the pharmaceutical composition prepared in Comparative Example 8, carried out in Experimental Example 8.

Figure 15 is a graph showing the results of a dissolution test of the pharmaceutical compositions of the present invention prepared in Examples 16 and 17, carried out in Experimental Example 10.

Figure 16 is a graph showing time courses of plasma concentrations of mirabegron in fasted and fed states, in a pharmacokinetics test (dogs) of the pharmaceutical composition of the present invention prepared in Example 16, carried out in Experimental Example 11.

Figure 17 is a graph showing the results of a dissolution test of a suspension prepared by dispersing a liquid of the pharmaceutical composition of the present invention prepared in Example 16 to water, after being stored at a cool place (5°C) for 2 weeks (control: suspension before the storage), in a stability test of the liquid of the pharmaceutical composition of the present invention prepared in Example 16, carried out in Experimental Example 12.

Figure 18 is a graph showing the results of a dissolution test of a suspension prepared by dispersing a liquid of the pharmaceutical composition of the present invention prepared in Example 17 to water, after being stored at a cool place (5°C) for 2 weeks (control: suspension before the storage), in a stability test of the liquid of the pharmaceutical composition of the present invention prepared in Example 17, carried out in Experimental Example 12.

Figure 19 is a graph showing the results of a dissolution test of the pharmaceutical compositions of the present invention prepared in Examples 20 and 23, carried out in Experimental Example 13.

Figure 20 is a graph showing time courses of plasma concentrations of mirabegron in fasted and fed states, in a pharmacokinetics test (dogs) of the pharmaceutical composition of the present invention prepared in Example 23, carried out in Experimental Example 14.

Figure 21 is a graph showing the results of a dissolution test of a suspension prepared by dispersing a liquid of the pharmaceutical composition of the present invention prepared in Example 20 to water, after being stored at a cool place (5°C) for 2 weeks (control: suspension before the storage), in a stability test of the liquid of the pharmaceutical composition of the present invention prepared in Example 20, carried out in Experimental Example 15.

Figure 22 is a graph showing the results of a dissolution test of a suspension prepared by dispersing a liquid of the pharmaceutical composition of the present invention prepared in Example 23 to water, after being stored at a cool place (5°C) for 2 weeks (control: suspension before the storage), in a stability test of the liquid of the pharmaceutical composition of the present invention prepared in Example 23, carried out in Experimental Example 15.

## DESCRIPTION OF EMBODIMENTS

Hereinafter, the present invention will be explained in detail.

The term "alkyl sulfuric acid" as used herein means dodecyl sulfuric acid (lauryl sulfuric acid), tridecyl sulfuric acid, tetradecyl sulfuric acid (myristyl sulfuric acid), pentadecyl sulfuric acid, hexadecyl sulfuric acid (palmityl sulfuric acid), or dioctyl sulfuric acid; dodecyl sulfuric acid (lauryl sulfuric acid), tetradecyl sulfuric acid (myristyl sulfuric acid), or hexadecyl sulfuric acid (palmityl sulfuric acid) in an embodiment; and dodecyl sulfuric acid (lauryl sulfuric acid) in another embodiment.

Mirabegron is easily available in accordance with, for example, a method described in WO 99/20607, a method obvious to the those skilled in the art, or a modified method thereof. The acid addition salt of mirabegron may be prepared by methods described in the Examples below, a modified method thereof, or a method utilizing a conventional salt

formation reaction.

Embodiments of the acid addition salt of alkyl sulfuric acid and mirabegron of the present invention, and the pharmaceutical composition of the present invention are shown as follows:

(1) An acid addition salt of alkyl sulfuric acid and mirabegron, which is an acid addition salt of an acid selected from the group consisting of dodecyl sulfuric acid, tridecyl sulfuric acid, tetradecyl sulfuric acid, pentadecyl sulfuric acid, hexadecyl sulfuric acid, and dioctyl sodium sulfate with mirabegron. In another embodiment, an acid addition salt of alkyl sulfuric acid and mirabegron, which is an acid addition salt of an acid selected from the group consisting of dodecyl sulfuric acid, tetradecyl sulfuric acid, and hexadecyl sulfuric acid with mirabegron. In still another embodiment, an acid addition salt of alkyl sulfuric acid and mirabegron, which is an acid addition salt selected from the group consisting of mirabegron didodecyl sulfate, mirabegron ditetradecyl sulfate, and mirabegron dihexadecyl sulfate. In still another embodiment, an acid addition salt of alkyl sulfuric acid and mirabegron, which is mirabegron didodecyl sulfate. In still another embodiment, an acid addition salt of alkyl sulfuric acid and mirabegron, which is mirabegron ditetradecyl sulfate. In still another embodiment, an acid addition salt of alkyl sulfuric acid and mirabegron, which is mirabegron dihexadecyl sulfate. In still another embodiment, an acid addition salt of alkyl sulfuric acid and mirabegron, which is an acid addition salt selected from the group consisting of mirabegron monododecyl sulfate, mirabegron monotetradecyl sulfate, and mirabegron monohexadecyl sulfate. In still another embodiment, an acid addition salt of alkyl sulfuric acid and mirabegron, which is mirabegron monododecyl sulfate. In still another embodiment, an acid addition salt of alkyl sulfuric acid and mirabegron, which is mirabegron monotetradecyl sulfate. In still another embodiment, an acid addition salt of alkyl sulfuric acid and mirabegron, which is mirabegron monohexadecyl sulfate.

(2) A pharmaceutical composition, which is a pharmaceutical composition for the treatment of overactive bladder. In another embodiment, a pharmaceutical composition, which is a pharmaceutical composition for the treatment of urinary urgency accompanied by overactive bladder. In still another embodiment, a pharmaceutical composition, which is a pharmaceutical composition for the treatment of urinary frequency accompanied by overactive bladder. In still another embodiment, a pharmaceutical composition, which is a pharmaceutical composition for the treatment of urinary incontinence accompanied by overactive bladder.

(3) A pharmaceutical composition, comprising an acid addition salt of alkyl sulfuric acid and mirabegron, and a water-soluble polymer. In another embodiment, a pharmaceutical composition, comprising an acid addition salt of alkyl sulfuric acid and mirabegron, and a water-insoluble substance. In still another embodiment, a pharmaceutical composition, comprising an acid addition salt of alkyl sulfuric acid and mirabegron, and a water-insoluble polymer.

(4) A compound or a pharmaceutical composition, which is a combination of two or more embodiments described in the above-mentioned (1) to (3).

The above-mentioned embodiments include various hydrates, solvates, and crystal polymorphism of acid addition salts of alkyl sulfuric acid and mirabegron, and pharmaceutical compositions containing the same. Further, the embodiments include compounds labeled with various radioactive or non-radioactive isotopes, and pharmaceutical compositions containing the same.

With respect to the acid addition salt of alkyl sulfuric acid and mirabegron, the molar ratio of mirabegron to alkyl sulfuric acid (mirabegron:alkyl sulfuric acid) is approximately 1:1 to 1:2 and, in another embodiment, 1:1, 1:2, or a combination thereof.

The expression "suspended or dispersed in a solvent and stored" as used herein means to be stored in a state where it is uniformly suspended or dispersed in a pharmaceutically acceptable solvent. For example, it means that the pharmaceutical composition of the present invention is stored under various temperature conditions (at room temperature, at 25°C, or at a cool place (5°C)) for two weeks or more. In another embodiment, it means that it is stored at room temperature, at 25°C, or at a cool place (5°C) for two weeks or for a month.

Examples of a solvent for suspension or a solvent for dispersion include water, a xanthan gum solution, a solution of polyvinyl alcohol, glycerin, propylene glycol, and commercially available swallowable agents. The xanthan gum solution may be prepared by adding xanthan gum (manufactured by Nitta Gelatin Inc., product name: VS-900; the same was used hereinafter) to water at a concentration of 1 W/W%.

The expression "the dissolution or leakage of mirabegron is reduced or inhibited" as used herein means when the pharmaceutical composition of the present invention, which was dispersed in a solvent and has been stored, is subjected to a dissolution test in accordance with the dissolution test, method 2 (paddle method) described in the Japanese Pharmacopoeia, using 900 mL of an appropriate test solution (for example, the second fluid (JP2) described in the dissolution test of the Japanese Pharmacopoeia), for example, at a paddle rotation speed of 50-200 rpm, the change in dissolution rate is 15% or less at the maximum in comparison with the value before the storage. The change in dissolution rate is 10% or less in another embodiment, 5% or less in still another embodiment, and 3% or less in still another embodiment.

Hereinafter, the pharmaceutical composition of the present invention will be explained.

The expression "the change in pharmacokinetics caused by the presence or absence of food intake is reduced" as used herein means that the change in maximum blood drug concentration (Cmax) or area under a blood drug concentration versus time curve (AUC) when administered after eating a meal, in comparison with a Cmax or AUC when administered in a fasted state, is approximately 30% or less, approximately 20% or less, and approximately 10% or less. The rates of decrease of Cmax and AUC are calculated by the following equations:

$$Rd(Cmax) = [1-Cmax(Fed)/Cmax(Fasted)]\times 100$$

$$Rd(AUC) = [1-AUC(Fed)/AUC(Fasted)]\times 100$$

Rd(Cmax): Rate of decrease of Cmax (%)
Cmax(Fed): Cmax in administration after food intake
Cmax(Fasted): Cmax in administration in the fasted state

Rd(AUC): Rate of decrease of AUC (%)
AUC(Fed): AUC in administration after food intake
AUC(Fasted): AUC in administration in the fasted state

The "pharmaceutical composition in which the change in pharmacokinetics caused by the presence or absence of food intake is reduced" may be evaluated, for example, in accordance with an in vitro test method. As such an in vitro test method, when a dissolution test is carried out, as an embodiment, in accordance with the dissolution test, method 2 (paddle method) described in the Japanese Pharmacopoeia, using 900 mL of the second fluid (JP2) described in the dissolution test of the Japanese Pharmacopoeia as the test solution, at a paddle rotation speed of 50-200 rpm, the dissolution rate of mirabegron after 30 minutes from the beginning of the test is approximately less than 85%. In another embodiment, the dissolution rate of mirabegron after 1.5 hours from the beginning of the test is approximately 70% or less. In still another embodiment, the dissolution rate of mirabegron after 1.5 hours is approximately 70% or less, and the dissolution rate of mirabegron after 10 hours is approximately 60% or more and approximately 100% or less. In still another embodiment, the dissolution rate of mirabegron after 1.5 hours is approximately 70% or less, and the dissolution rate of mirabegron after 10 hours is approximately 70% or more and approximately 100% or less.

The expression "bitterness is inhibited or reduced" as used herein means a state where the bitterness of the drug can be inhibited or reduced at a drug concentration less than a specific drug concentration. For example, when the pharmaceutical composition of the present invention is added to $10^{-1}$ mol/L hydrochloric acid, $10^{-3}$ mol/L hydrochloric acid, or purified water, and shaked or stirred at room temperature for a day, and the drug concentration contained in the supernatant derived from each liquid is measured, the drug concentration is 0.1 mg/mL or less in an embodiment, 0.05 mg/mL or less in another embodiment, and 0.03 mg/mL or less in still another embodiment, as the index.

The base for modified release, which is used in the present invention, is not limited, so long as it is, for example, a base capable of reducing the change in pharmacokinetics of mirabegron regardless of the presence or absence of food intake; a base capable of reducing or inhibiting the dissolution or leakage of mirabegron when being suspended or dispersed in a solvent or during its storage; a base which does not cause bitterness even when it is used as liquids, suspensions, or emulsions; or a base capable of inhibiting the mirabegron concentration at a concentration equal to or less than a concentration which is assumed to be the above-mentioned index. Examples of such bases include water-soluble polymers and water-insoluble substances. In another embodiment, the base for modified release is a water-insoluble polymer.

Examples of the water-soluble polymers include, for example, polyethylene oxide, hypromellose having a molecular weight of 1,000 to 4,000,000, hydroxymethyl cellulose, hydroxyethyl cellulose, hydroxypropyl cellulose having a molecular weight of 2,000 to 2,000,000, carboxyvinyl polymers, chitosans, mannans, galactomannans, xanthans, carageenans, amylose, alginic acid, salts and derivatives thereof, pectin, polyacid anhydrides, polyamino acids, poly(methylvinyl ether/maleic anhydride)polymers, polyvinyl alcohols, glucans, scleroglucans, carboxymethyl cellulose and derivatives thereof, methyl cellulose, or conventional water-soluble cellulose derivatives. The water-soluble polymers are hypromellose having a molecular weight of 1,000 to 2,000,000, or carboxyvinyl polymers of 3,000 to 45,000 cps (0.5% aqueous solution at 25°C) in another embodiment, and hypromellose having a molecular weight of 10,000 to 1,000,000, or carboxyvinyl polymers of 4,000 to 40,000 cps (0.5% aqueous solution at 25°C) in still another embodiment.

The content of the water-soluble polymer is 5 W/W% to 95 W/W% per formulation unit, 10 W/W% to 90 W/W% in another embodiment, and 30 W/W% to 85 W/W% in still another embodiment.

Examples of polyethylene oxide (hereinafter sometimes referred to as PEO) include product names, Polyox WSR-308 [average molecular weight: 8,000,000, viscosity: 10,000-15,000 mPa·s (1% aqueous solution at 25°C)], Polyox WSR-303 [average molecular weight: 7,000,000, viscosity: 7,500-10,000 mPa·s (1% aqueous solution at 25°C)], Polyox WSR Coagulant [average molecular weight: 5,000,000, viscosity: 5,500-7,500 mPa·s (1% aqueous solution at 25°C)], Polyox WSR-301 [average molecular weight: 4,000,000, viscosity: 1,650-5,500 mPa·s (1% aqueous solution at 25°C)], Polyox WSR-N-60K [average molecular weight: 2,000,000, viscosity: 2,000-4,000 mPa·s (2% aqueous solution at 25°C)], Polyox WSR-N-12K [average molecular weight: 1,000,000, viscosity: 400-800 mPa·s (2% aqueous solution at 25°C)], Polyox WSR-1105 [average molecular weight: 900,000, viscosity: 8,800-17,600 mPa·s (5% aqueous solution at 25°C)], Polyox WSR-205 [average molecular weight: 600,000, viscosity: 4,500-8,800 mPa·s (5% aqueous solution at 25°C)], Polyox WSR-N-750 [average molecular weight: 300,000, viscosity: 600-1200 mPa·s (5% aqueous solution at 25°C)], Polyox WSR-N-80 [average molecular weight: 200,000, viscosity: 55-90 mPa·s (5% aqueous solution at 25°C)], and Polyox WSR-N-10 [average molecular weight: 100,000, viscosity: 12-50 mPa·s (5% aqueous solution at 25°C)](DOW).

Examples of hypromellose (hereinafter sometimes referred to as HPMC) include product names, Metolose 90SH50000 [viscosity in a 2% aqueous solution at 20°C: 2,900-3,900 mPa·s], Metolose SB-4 (viscosity in a 2% aqueous solution at 20°C: approximately 4mPa·s), TC-5RW (viscosity in a 2% aqueous solution at 20°C: approximately 6mPa·s), TC-5S (viscosity in a 2% aqueous solution at 20°C: approximately 15mPa·s), TC-5R (viscosity in a 2% aqueous solution at 20°C: approximately 6mPa·s), TC-5M (viscosity in a 2% aqueous solution at 20°C: approximately 4.5mPa·s), TC-5E (viscosity in a 2% aqueous solution at 20°C: approximately 3mPa·s), Metolose 60SH-50 (viscosity in a 2% aqueous solution at 20°C: approximately 50 mPa·s), Metolose 65SH-50 (viscosity in a 2% aqueous solution at 20°C: approximately 50 mPa·s), Metolose 90SH-100 (viscosity in a 2% aqueous solution at 20°C: approximately 100 mPa·s), Metolose 90SH-100SR (viscosity in a 2% aqueous solution at 20°C: approximately 100 mPa·s), Metolose 65SH-400 (viscosity in a 2% aqueous solution at 20°C: approximately 400 mPa·s), Metolose 90SH-400 (viscosity in a 2% aqueous solution at 20°C: approximately 400 mPa·s), Metolose 65SH-1500 (viscosity in a 2% aqueous solution at 20°C: approximately 1,500 mPa·s), Metolose 60SH-4000 (viscosity in a 2% aqueous solution at 20°C: approximately 4,000 mPa·s), Metolose 65SH-4000 (viscosity in a 2% aqueous solution at 20°C: approximately 4,000 mPa·s), Metolose 90SH-4000 (viscosity in a 2% aqueous solution at 20°C: approximately 4,000 mPa·s), Metolose 90SH-4000SR (viscosity in a 2% aqueous solution at 20°C: approximately 4,000 mPa·s), Metolose 90SH-15000 (viscosity in a 2% aqueous solution at 20°C: approximately 15,000 mPa·s), Metolose 90SH-15000SR (viscosity in a 2% aqueous solution at 20°C: approximately 15,000 mPa·s), and Metolose 90SH-30000 (viscosity in a 2% aqueous solution at 20°C: approximately 30,000 mPa·s) (Shin-Etsu Chemical Co., Ltd.).

Examples of hydroxypropyl cellulose (hereinafter sometimes referred to as HPC) include product names, HPC-SSL (viscosity in a 2% aqueous solution at 20°C: 2.0-2.9 mPa·s), HPC-SL (viscosity in a 2% aqueous solution at 20°C: 3.0-5.9 mPa·s), HPC-L (viscosity in a 2% aqueous solution at 20°C: 6.0-10.0 mPa·s), HPC-M (viscosity in a 2% aqueous solution at 20°C: 150-400 mPa·s), and HPC-H (viscosity in a 2% aqueous solution at 20°C: 1,000-4,000 mPa·s)(Nippon Soda Co., Ltd.).

Examples of methylcellulose (hereinafter sometimes referred to as MC) include product names, Metolose SM15 (viscosity in a 2% aqueous solution at 20°C: approximately 15 mPa·s), Metolose SM25 (viscosity in a 2% aqueous solution at 20°C: approximately 25 mPa·s), Metolose SM100 (viscosity in a 2% aqueous solution at 20°C: approximately 100 mPa·s), Metolose SM400 (viscosity in a 2% aqueous solution at 20°C: approximately 400 mPa·s), Metolose SM1500 (viscosity in a 2% aqueous solution at 20°C: approximately 1,500 mPa·s), and Metolose SM4000 (viscosity in a 2% aqueous solution at 20°C: approximately 4,000 mPa·s) (Shin-Etsu Chemical Co., Ltd.).

Examples of carboxymethyl cellulose sodium (hereinafter sometimes referred to as CMCNa) include product names, Sunrose F-30MC [viscosity: 250-350 mPa·s (1% aqueous solution at 25°C)], Sunrose F-150MC [average molecular weight: 200,000, viscosity: 1,200-1,800 mPa·s (1% aqueous solution at 25°C)], Sunrose F-600MC [viscosity: 6,000-8,000 mPa·s (1% aqueous solution at 25°C)], Sunrose F-1000MC [average molecular weight: 420,000, viscosity: 8,000-12,000 mPa·s (the same)], Sunrose F-1400MC [viscosity: 12,000-15,000 mPa·s (1% aqueous solution at 25°C)], and Sunrose F-300MC [average molecular weight: 300,000, viscosity: 2,500-3,000 mPa·s (the same)](Nippon Paper Chemicals Co., Ltd.).

Examples of hydroxyethyl cellulose (hereinafter sometimes referred to as HEC) include product names, HEC DAICEL SE850 [average molecular weight: 1,480,000, viscosity: 2,400-3,000 mPa·s (1% aqueous solution at 25°C)], and HEC DAICEL SE900 [average molecular weight: 1,560,000, viscosity: 4,000-5,000 mPa·s (1% aqueous solution at 25°C)](Daicel chemical Industries, Ltd.).

Examples of carboxyvinyl polymers include product names, Carbopol 71G (viscosity: 4,000-11,000 mPa·s), Carbopol 971P (viscosity: 4,000-11,000 mPa·s), Carbopol 981 (viscosity: 4,000-10,000 mPa·s), Carbopol 941 (viscosity: 4,000-10,000 mPa·s), Carbopol 934 (viscosity: 30,500-39,400 mPa·s), and Carbopol 934P (viscosity: 29,400-39,400 mPa·s)(B.F.Goodrich Chemical).

These water-soluble polymers may be used alone, or as an appropriate combination of two or more thereof. A combination of different lots may be used.

The content of the water-soluble polymer is not limited, so long as it is an amount to the extent, for example, that the blood concentration profile of the drug does not affect the change in pharmacokinetics caused by the presence or absence of food intake; that when the pharmaceutical composition is a solid, and suspended or dispersed in a solvent, or when it is suspended or dispersed in a solvent, and stored, the dissolution or leakage of mirabegron can be reduced or inhibited; that bitterness is not caused even when it is used as liquids, suspensions, or emulsions; or that the mirabegron concentration can be inhibited at a concentration equal to or less than a concentration which is assumed to be the above-mentioned index. The content of the water-soluble polymer is, for example, 1 W/W% or more and 70 W/W% or less with respect to the total weight of the formulation, and 3 W/W% or more and 70 W/W% or less in another embodiment. The content of the water-soluble polymer is 5 W/W% or more and 70 W/W% or less with respect to the total weight of the formulation, 10 W/W% or more and 60 W/W% or less in another embodiment, and 10 W/W% or more and 40 W/W% or less in still another embodiment. The content of the water-soluble polymer with respect to the weight of the drug is 0.1 W/W% or more and 1000 W/W% or less, 1 W/W% or more and 500 W/W% or less in another embodiment, and 5 W/W% or more and 300 W/W% or less in still another embodiment.

A water-soluble polymer of which the viscosity (before mixing) is beyond the specific range can be used as an appropriate combination with one or more other water-soluble polymers, in case that the mixture obtained by mixing these plural polymers has a viscosity (as measured before the use) within the specific range.

In a case that the water-soluble polymer is used as the base for modified release, the pharmaceutical composition of the present invention may contain an additive which ensures penetration of water into the formulation (hydrophilic base).

In the additive which ensures penetration of water into the pharmaceutical composition of the present invention (hydrophilic base), the amount of water necessary to dissolve 1 g of the hydrophilic base at 20±5°C is 10 mL or less, 6 mL or less in another embodiment, 5 mL or less in still another embodiment, and 4 mL or less in still another embodiment. When the hydrophilic base has a high solubility to water, the effect that allows water to penetrate into the formulation is high.

Examples of the hydrophilic base include water-soluble polymers, such as polyethylene glycol [PEG: for example, product names PEG 400, PEG 1500, PEG 4000, PEG 6000, and PEG 20000 (NOF Corporation)], polyvinyl pyrrolidone (PVP: for example, product name PVP K30 (BASF), and the like; sugar alcohols, such as D-mannitol, D-sorbitol, xylitol, and the like; saccharides, such as lactose, sucrose, anhydrous maltose, D-fructose, dextran (for example, Dextran 40), glucose, and the like; surfactants, such as polyoxyethylene hydrogenated castor oil [HCO: for example, Cremophor RH40 (BASF), HCO-40, HCO-60 (Nikko Chemicals)], polyoxyethylene polyoxypropylene glycol [for example, Pluronic F68 (ADEKA Corporation and the like)], polyoxyethylene sorbitan higher fatty acid esters [Tween: for example, Tween 80 (Kanto Chemical)], and the like; salts, such as sodium chloride, magnesium chloride, and the like; organic acids, such as citric acid, tartaric acid, and the like; amino acids, such as glycine, β-alanine, lysine hydrochloride, and the like; and aminosaccharides, such as meglumine and the like.

As another embodiment, PEG, PVP, D-mannitol, D-sorbitol, xylitol, lactose, sucrose, anhydrous maltose, D-fructose, dextran, glucose, polyoxyethylene polyoxypropylene glycol, sodium chloride, magnesium chloride, citric acid, tartaric acid, glycine, *β*-alanine, lysine hydrochloride, or meglumine may be used. As still another embodiment, PEG, PVP, D-mannitol, lactose, sucrose, sodium chloride, polyoxyethylene polyoxypropylene glycol, or the like may be used.

These hydrophilic bases may be used alone, or as an appropriate combination of two or more thereof.

The content of the hydrophilic base is not limited, so long as it is an amount, for example, capable of controlling the release of the drug to the extent that the change in pharmacokinetics caused by the presence or absence of food intake is not affected; capable of reducing or inhibiting the dissolution or leakage of mirabegron when the pharmaceutical composition is a solid, and suspended or dispersed in a solvent, or when it is suspended or dispersed in a solvent, and stored; capable of inhibiting bitterness even when it is used as liquids, suspensions, or emulsions; or capable of inhibiting the drug concentration at a concentration equal to or less than a concentration which is assumed to be the above-mentioned index and which does not cause bitterness. The content of the hydrophilic base is, for example, 5 W/W% or more and 75 W/W% or less with respect to the total weight of the formulation, 5 W/W% or more and 70 W/W% or less in another embodiment, and 20 W/W% or more and 60 W/W% or less in still another embodiment.

Examples of the water-insoluble substances include water-insoluble polymers ,wax-like substances and the like.

Examples of the water-insoluble polymers include water-insoluble cellulose ethers, such as ethylcellulose (for example, product name: Ethocel STD 10, manufactured by Dow Chemical; an aqueous dispersion of ethylcellulose (for example, product name: Aquacoat ECD, manufactured by FMC)); and water-insoluble acrylate copolymers, such as ethyl acrylate/methyl methacrylate/trimethylammoniumethyl methacrylate chloride copolymers (for example, product names: Eudragit RS100 and Eudragit RS30D, manufactured by EVONIK Roehm), ethyl acrylate/methyl methacrylate copolymers (for example, product name: Eudragit NE30D, manufactured by EVONIK Roehm) and the like.

Examples of the wax-like substances include solid fats, such as hydrogenated castor oil, hydrogenated coconut oil, and beef tallow; higher fatty acids, such as stearic acid, lauric acid, myristic acid, and palmitic acid; and higher alcohols, such as cetyl alcohol and stearyl alcohol.

Preferred water-insoluble substances are ethylcellulose, ethyl acrylate/methyl methacrylate/trimethylammoniumethyl methacrylate chloride copolymers, and ethyl acrylate/methyl methacrylate copolymers. Ethylcellulose, or ethyl

acrylate/methyl methacrylate/trimethylammoniumethyl methacrylate chloride copolymers may be used in another embodiment, and ethylcellulose may be used in still another embodiment.

In a case that the water-insoluble polymer is used as the base for modified release, the pharmaceutical composition of the present invention may contain a plasticizer. The plasticizer is not limited, so long as it improves the plasticity of the water-insoluble polymer. Examples of the plasticizer include triethyl citrate, PEG400, PEG600, PEG1500, PEG4000, PEG6000, triacetin, glycerin, glycerol monostearate, acetylated monoglyceride and the like.

The content of the water-insoluble substance is not limited, so long as it is an amount, for example, capable of reducing the change in pharmacokinetics of mirabegron regardless the presence or absence of food intake; capable of reducing or inhibiting the dissolution or leakage of mirabegron, when it is suspended or dispersed in a solvent, or during its storage; capable of inhibiting bitterness even when it is used as liquids, suspensions, or emulsions; or capable of inhibiting the drug concentration at a concentration equal to or less than a concentration which is assumed to be the above-mentioned index and which does not cause bitterness. More particularly, the content of the water-insoluble substance is 0.1 W/W% or more and 1000 W/W% or less, with respect to the weight of the acid addition salt of alkyl sulfuric acid and mirabegron, in an embodiment. Furhter, the content of the water-insoluble substance is 0.1 W/W% or more and 300 W/W% or less in another embodiment,50 W/W% or more and 300 W/W% or less in still another embodiment, and 50 W/W% or more and 150 W/W% or less in still another embodiment.

In the pharmaceutical composition of the present invention, the structure of the acid addition salt of alkyl sulfuric acid and mirabegron, and the base for modified release is not limited in each of uniform embodiments or non-uniform embodiments, so long as it is a structure, for example, capable of reducing the change in pharmacokinetics of mirabegron regardless of the presence or absence of food intake; capable of reducing or inhibiting the dissolution or leakage of mirabegron, when the pharmaceutical composition is a solid, and suspended or dispersed in a solvent, or when it is suspended or dispersed in a solvent, and stored; capable of inhibiting bitterness even when it is used as liquids, suspensions, or emulsions; or capable of inhibiting the drug concentration at a concentration equal to or less than a concentration which is assumed to be the above-mentioned index and which does not cause bitterness. Examples of such structures include an embodiment in which the acid addition salt of alkyl sulfuric acid and mirabegron is coated and/or granulated with the base for modified release; an embodiment in which the acid addition salt of alkyl sulfuric acid and mirabegron is mixed with the base for modified release; an embodiment in which capsules are filled with the pharmaceutical composition comprising the acid addition salt of alkyl sulfuric acid and mirabegron, and the base for modified release; an embodiment in which the acid addition salt of alkyl sulfuric acid and mirabegron, and the base for modified release are suspended or dissolved in a solvent; and an embodiment in which tablets containing the acid addition salt of alkyl sulfuric acid and mirabegron are coated with the base for modified release.

These embodiments of the pharmaceutical composition may be used alone, or as an appropriate combination of two or more.

In the pharmaceutical composition of the present invention, various pharmaceutical additives may be further used, if desired. Such pharmaceutical additives are not limited, so long as they are pharmaceutically acceptable and pharmacologically acceptable. Examples of the pharmaceutical additives include binders, stabilizers, disintegrating agents, acidulants, foaming agents, artificial sweeteners, lubricants, coloring agents, buffers, antioxidants, solubilizing agents, preservatives, flavors, perfumes, suspending agents, dispersing agents, thickeners, wetting agents, defoaming agents, solvents and the like.

Examples of the binders include gum arabic, hypromellose, hydroxypropyl cellulose, hydroxyethyl cellulose and the like.

Examples of the stabilizers include yellow ferric oxide, red ferric oxide, black iron oxide and the like.

Examples of the disintegrating agents include corn starch, potato starch, carmellose calcium, carmellose sodium, low-substituted hydroxypropyl cellulose and the like.

Examples of the acidulants include citric acid, tartaric acid, malic acid and the like.

Examples of the foaming agents include sodium bicarbonate and the like.

Examples of the artificial sweeteners include saccharin sodium, dipotassium glycyrrhizin, aspartame, stevia, thaumatin and the like.

Examples of the lubricants include magnesium stearate, calcium stearate, sucrose fatty acid ester, polyethylene glycol, talc, stearic acid and the like.

Examples of the coloring agents include food yellow No. 4, food yellow No. 5, food red No. 3, food red No. 102, food blue No. 3 and the like.

Examples of the buffers include citric acid, succinic acid, fumaric acid, tartaric acid, ascorbic acid, or salts thereof, glutamic acid, glutamine, glycine, aspartic acid, alanine, arginine, or salts thereof, magnesium oxide, or salts thereof, zinc oxide, magnesium hydroxide, phosphoric acid, boric acid, or salts thereof and the like.

Examples of the antioxidants include ascorbic acid, dibutyl hydroxy toluene, and propyl gallate.

Examples of the solubilizing agents include polysorbate 80, sodium lauryl sulfate, polyoxyethylene hydrogenated castor oil and the like.

Examples of the preservatives include methyl parahydroxybenzoate, ethyl parahydroxybenzoate, propyl parahydroxybenzoate, butyl parahydroxybenzoate, benzoic acid, benzyl alcohol, sorbic acid, acetic acid, or salts thereof and the like.

Examples of the flavors include sugars or sugar alcohols, such as sucrose, fructose, lactose, sorbitol, mannitol, xylitol and the like; sweeteners, such as aspartame, acesulfame potassium, sucralose and the like; and the like.

Examples of the perfumes include lemon, lemon lime, orange, menthol, strawberry, banana, raspberry, bubble gum flavor and the like.

Examples of the suspending agents, dispersing agents, or thickeners include locust bean gum, guar gum, pullulan, xanthan gum, carrageenan, tragacanth gum, dextrin, pectin, gelatin and the like. In addition to these additives, a non-ionic substance may be added, if necessary.

Examples of the wetting agents include polyoxyethylene sorbitan fatty acid esters, such as polysorbate 80 and Arlacel 83; polyoxyethylene hydrogenated castor oil, such as HCO-50 and the like; and surfactants, such as sugar esters and the like; and the like.

Examples of the defoaming agents include simethicone, dimethicone and the like.

Examples of the solvents include water, a xanthan gum solution, a solution of polyvinyl alcohol, glycerin, propylene glycol, commercially available swallowable agents and the like.

These pharmaceutical additives may be appropriately added alone or as a combination of two or more, at appropriate amounts.

With the contents of the pharmaceutical additives, each pharmaceutical additive may be used within an amount by which the desired effects of the present invention can be achieved.

The pharmaceutical composition may be administered by oral administration, such as tablets, orally disintegrating tablets, pills, capsules, granules, powders, liquids or the like, or by parenteral administration, such as intra-articular injections, intravenous injections, intramuscular injections, suppositories, eye drops, eye ointments, transdermal solutions, ointments, transdermal patches, transmucosal solutions, transmucosal patches, inhalants or the like. In an embodiment, orally disintegrating tablets, granules, powders, liquids, suspensions, or emulsions may be orally administered, and in another embodiment, orally disintegrating tablets, liquids, suspensions, or emulsions may be orally administered. In still another embodiment, liquids, suspensions, or emulsions may be orally administered. In still another embodiment, orally disintegrating tablets may be orally administered.

In case of general oral administration, the daily dose (as the amount of mirabegron) is appropriately approximately 0.001 to 100 mg/kg, preferably 0.1 to 30 mg/kg, and more preferably 0.1 to 10 mg/kg, which is administered once or divided into two to four doses per day. In case of intravenous administration, the daily dose (as the amount of mirabegron) is appropriately approximately 0.0001 to 10 mg/kg, which is administered once or divided into plural doses per day. In case of transmucosal agents, the daily dose (as the amount of mirabegron) is approximately 0.001 to 100 mg/kg, which is administered once or divided into plural doses per day. The dose of mirabegron may be appropriately selected in accordance with symptom, age, sex, and the like of the patient.

The acid addition salt of alkyl sulfuric acid and mirabegron of the present invention may be used together with various agents for the treatment or prevention of the above-mentioned diseases to which the acid addition salt of alkyl sulfuric acid and mirabegron is considered effective. In this combined administration, these may be administered simultaneously, or separately and sequentially, or at a desired interval(s). A formulation for simultaneous administration may be a single dosage form, or separate dosage forms.

Hereinafter, the process of manufacturing the pharmaceutical composition comprising the acid addition salt of alkyl sulfuric acid and mirabegron, and the base for modified release will be explained.

Mirabegron and alkyl sulfuric acid are respectively dissolved in each solvent. The solvents are not limited, so long as mirabegron and alkyl sulfuric acid can be dissolved. Examples of the solvents include water, hydrochloric acid, phosphoric acid, organic solvents such as ethanol or methanol, and a mixture thereof. It is not necessary that mirabegron and alkyl sulfuric acid are completely dissolved.

Next, the resulting mirabegron solution is mixed with the resulting alkyl sulfuric acid solution. Although the mixing conditions are not limited, the mixing may be carried out, for example, by adding the alkyl sulfuric acid solution to the mirabegron solution, or by adding the mirabegron solution to the alkyl sulfuric acid solution.

Alternatively, the mixing may be carried out by adding a solvent capable of dissolving mirabegron and alkyl sulfuric acid, such as water or hydrochloric acid, to a mixture prepared by mixing mirabegron powder with alkyl sulfuric acid powder.

After such a mixture liquid is prepared, a step of collecting a precipitate by filtration may be carried out.

Next, the resulting precipitate or the mixture liquid is dried. A method for drying is not limited, so long as the precipitate or the mixture liquid can be dried. Examples of the drying method include methods using a ventilation dryer, a vacuum dryer, a fluidized bed granulator, or a spray dryer. The drying temperature is, for example, 40 to 60°C.

The base for modified release is further added to the resulting dried product. The base for modified release is previously dispersed or dissolved in water or an organic solvent, and added to the dried product. A method for addition

is not limited, but examples thereof include methods using a fluidized bed granulator, a spray dryer, an agitation granulator, or a compact agitation granulator. After the addition of the base for modified release, a drying step may be carried out. Further, a step of controlling temperature and/or humidity may be carried out.

## EXAMPLES

Hereinafter, a process of manufacturing the acid addition salt of alkyl sulfuric acid and mirabegron, and the pharmaceutical composition containing thereof of the present invention will be explained on the basis of the Examples. The present invention is not limited to the acid addition salt and the pharmaceutical composition described in the following Examples. Further, the process of manufacturing the acid addition salt of alkyl sulfuric acid and mirabegron, and the pharmaceutical composition is not limited to the following production methods disclosed as concrete working examples, and the acid addition salt of alkyl sulfuric acid and mirabegron, and the pharmaceutical composition may be produced by a combination of these production methods, or a method which is obvious for those skilled in the art.

### Example 1

Liquid A was prepared by dissolving 122 g of mirabegron (manufactured by Astellas Pharma Inc.; the same was used hereinafter) in 6100 g of 0.1 mol/L hydrochloric acid. Liquid B was prepared by dissolving 178 g of sodium lauryl sulfate (manufactured by Cognis, product name: Texapon K12; the same was used hereinafter) in 6100 g of purified water. The resulting liquid B was added to liquid A at 160 g/min, and the mixture was stirred and mixed at a paddle speed of 170 to 200 rpm to obtain a precipitate. The resulting precipitate was filtered and collected using a 0.45 μm HA filter (manufactured by MILLIPORE; the same was used hereinafter), and tray-dried at 40°C for 24 hours followed by dried under reduced pressure at 60°C for 12 hours, to obtain powder of an acid addition salt of mirabegron and lauryl sulfuric acid of the present invention at a molar ratio of 1:2 (hereinafter sometimes abbreviated to as mirabegron lauryl sulfate).

The resulting powder was subjected to a $^1$H-NMR measurement (DMSO-$d_6$ solvent) to identify its components. Signals derived from sodium lauryl sulfate and mirabegron were observed, and it was confirmed that the powder was composed of both compounds. Salt formation by protonating the amino group of mirabegron was observed. The ratio of mirabegron to lauryl sulfuric acid was calculated from an integral ratio of $^1$H-NMR as follows. It was estimated from the integral ratio of a $\delta$0.85 (3H) peak derived from the methyl group at the terminus of lauryl sulfuric acid to $\delta$4.92 (1H) peak derived from the proton of the thiazole group of mirabegron that the acid addition salt had the structure in which 2 mol of lauryl sulfuric acid was added with respect to 1 mol of mirabegron.

### Example 2

Liquid A was prepared by dissolving 1g of mirabegron in 0.1 mol/L hydrochloric acid at room temperature. Liquid B was prepared by dissolving 1.60 g of sodium myristyl sulfate (manufactured by Nikko Chemicals, product name: NIKKOL SMS) in purified water previously heated at 50°C. The resulting liquid B was added to liquid A while heating at 50°C to obtain a precipitate. The resulting precipitate was filtered and collected using a 0.45 μm HA filter, and tray-dried at 40°C for 12 hours to obtain an acid addition salt of mirabegron and myristyl sulfuric acid of the present invention at a molar ratio of 1:2.

### Example 3

Liquid A was prepared by dissolving 1 g of mirabegron in 0.1 mol/L hydrochloric acid at room temperature. Liquid B was prepared by dissolving 1.74 g of sodium cetyl sulfate (manufactured by Nikko Chemicals Co., Ltd., product name: NIKKOL SCS) in purified water previously heated at 70°C. The resulting liquid B was added to liquid A while heating at 70°C to obtain a precipitate. The resulting precipitate was filtered and collected using a 0.45 μm HA filter, and tray-dried at 40°C for 12 hours to obtain an acid addition salt of mirabegron and cetyl sulfuric acid of the present invention at a molar ratio of 1:2.

### Example 4

To a liquid prepared by dissolving 94.6 g of mirabegron in 472.8 mL of 0.1 mol/L hydrochloric acid, 138 g of sodium lauryl sulfate was stepwisely added, and the mixture was stirred and mixed at room temperature using a multipurpose mixer (manufactured by SHINAGAWA MACHINERY WORKS Co., Ltd., type 5DMR) at 62 rpm for 0.5 hour. The resulting mixture was tray-dried at 40°C for 42.5 hours to obtain an acid addition salt of mirabegron and lauryl sulfuric acid of the present invention at a molar ratio of 1:2.

Example 5

Liquid A was prepared by dissolving 25 g of mirabegron in 500 mL of methanol (manufactured by Kanto Chemical CO., INC.; the same was used hereinafter) and further adding thereto 65 mL of 0.1 mol/L hydrochloric acid. The pH of the resulting liquid A was 5.62. Liquid B was prepared by dissolving 18.25 g of sodium lauryl sulfate in 500 mL of purified water. The pH of the resulting liquid B was 9.69. The resulting liquid B was added to liquid A, and the mixture was stirred and mixed using a paddle at room temperature. The resulting precipitate was filtered and collected using a 0.45 μm HA filter, and dried under reduced pressure at 60°C for 12 hours, to obtain powder of an acid addition salt of mirabegron and lauryl sulfuric acid of the present invention at a molar ratio of 1:1.

The resulting powder was subjected to a $^1$H-NMR measurement (DMSO-$d_6$ solvent) to identify its components. Signals derived from sodium lauryl sulfate and mirabegron were observed, and it was confirmed that the powder was composed of both compounds. Salt formation by protonating the amino group of mirabegron was observed. The ratio of mirabegron to lauryl sulfuric acid was calculated from an integral ratio of $^1$H-NMR as follows. It was estimated from the integral ratio of a δ0.85 (3H) peak derived from the methyl group at the terminus of lauryl sulfuric acid to δ4.91 (1H) peak derived from the proton of the thiazole group of mirabegron that the acid addition salt had the structure in which 1 mol of lauryl sulfuric acid was added with respect to 1 mol of mirabegron.

Example 6

Liquid A was prepared by dissolving 205.5 g of mirabegron in 4110 mL of methanol and further adding thereto 529.4 mL of 0.1 mol/L hydrochloric acid. Liquid B was prepared by dissolving 150 g of sodium lauryl sulfate in 4110 mL of purified water. The resulting liquid B was added to liquid A, and the mixture was stirred and mixed using a paddle at room temperature, and dried under reduced pressure at 60°C for 7 days, to obtain an acid addition salt of mirabegron and lauryl sulfuric acid of the present invention at a molar ratio of 1:1.

Comparative Example 1

A drug aqueous solution of a Comparative Example was obtained by dissolving 500 mg of mirabegron in 200 mL of a 50 mmol/L phosphoric acid solution.

Comparative Example 2

A mixture of mirabegron and lauryl sulfuric acid (molar ratio = 1:2) of a Comparative Example was obtained by mixing 1.5 g of mirabegron with 2.19 g of sodium lauryl sulfate using a pestle in mortar.

Comparative Example 3

Liquid A was prepared by dissolving 1.5 g of mirabegron in 75 mL of 0.1 mol/L hydrochloric acid at room temperature. Liquid B was prepared by dissolving 3.29 g of sodium lauryl sulfate in 75 mL of purified water. The resulting liquid B was added to liquid A at room temperature, and dried at 60°C to obtain a mixture of mirabegron and lauryl sulfuric acid (molar ratio = 1:3) of a Comparative Example.

Comparative Example 4

Liquid A was prepared by dissolving 1.5 g of mirabegron in 75 mL of 0.1 mol/L hydrochloric acid at room temperature. Liquid B was prepared by dissolving 5.48 g of sodium lauryl sulfate in 75 mL of purified water. The resulting liquid B was added to liquid A at room temperature, and dried at 60°C to obtain a mixture of mirabegron and lauryl sulfuric acid (molar ratio = 1:5) of a Comparative Example.

Comparative Example 5

Liquid A was prepared by dissolving 1 g of mirabegron in 50 mL of 0.1 mol/L hydrochloric acid at room temperature. Liquid B was prepared by dissolving 10.95 g of sodium lauryl sulfate in 150 mL of purified water. The resulting liquid B was added to liquid A at room temperature, and dried at 60°C to obtain a mixture of mirabegron and lauryl sulfuric acid (molar ratio = 1:15) of a Comparative Example.

The solubility and the like of each acid addition salt of alkyl sulfuric acid and mirabegron was confirmed by the following Experimental Examples.

Experimental Example 1

The drug concentration (solubility) of each compound to be assayed was measured in accordance with the following method.

The acid addition salts of the present invention prepared in Examples 1 to 3 (150 mg as the amount of mirabegron) were respectively added to $10^{-1}$ mol/L hydrochloric acid, $10^{-3}$ mol/L hydrochloric acid, and purified water, and the mixtures were shaked at a speed of 200±10 rpm at room temperature for 24 hours. After the shaking, the mixtures were centrifuged at 3000 rpm for 15 minutes, and the drug concentration in each supernatant was measured. Similarly, 150 mg of mirabegron was respectively added to $10^{-1}$ mol/L hydrochloric acid, $10^{-3}$ mol/L hydrochloric acid, and purified water, and the drug concentration in each supernatant was measured after 24 hours from the addition, under the same conditions as those of Examples 1 to 3.

The results of measuring drug concentrations in the acid addition salts of the present invention prepared in Examples 1 to 3 and mirabegron are shown in Table 1. The drug concentration could be inhibited in any solution of Examples 1 to 3, whereas mirabegron showed a high drug concentration in each solution.

[Table 1]

| Sample | Drug concentration in supernatant (mg/mL) | | |
|---|---|---|---|
| | $10^{-1}$ mol/L HCl | $10^{-3}$ mol/L HCl | Purified water |
| Mirabegron | 12.933 | 0.501 | 0.116 |
| Example 1 | 0.087 | 0.087 | 0.095 |
| Example 2 | 0.019 | 0.030 | 0.010 |
| Example 3 | 0.064 | 0.021 | 0.004 |

Experimental Example 2

The acid addition salts of the present invention prepared in Examples 1 to 3 (25 mg as the amount of mirabegron) were used to carry out a dissolution test in accordance with the dissolution test, method 2 (paddle method) described in the Japanese Pharmacopoeia. As the test solution, 900 mL of the second fluid (JP2) described in the disintegration test of the Japanese Pharmacopoeia was used. The paddle rotation speed was 150 rpm.

The measurement results of the dissolution test of the acid addition salts of the present invention prepared in Examples 1 to 3 are shown in Figure 1. The dissolution rates of Examples 1 to 3 were unexpectedly reduced in comparison with mirabegron.

Experimental Example 3

The acid addition salts of the present invention prepared in Examples 4 and 5 (25 mg as the amount of mirabegron) were used to carry out a dissolution test in accordance with the dissolution test, method 2 (paddle method) described in the Japanese Pharmacopoeia. As the test solution, 900 mL of the second fluid (JP2) described in the disintegration test of the Japanese Pharmacopoeia was used. The paddle rotation speed was 50 rpm. The same dissolution test was carried out using the mixtures prepared in Comparative Examples 2 to 5.

The results of the dissolution test are shown in Table 2 and Figure 2. The dissolution rates of Examples 4 and 5 were remarkably reduced in comparison with Comparative Examples 2 to 5. It was shown from these results that the dissolution rate could be reduced by converting mirabegron to an acid addition salt of mirabegron and lauryl sulfuric acid at a molar ratio of 1:1 to 1:2.

[Table 2]

| Sample | Dissolution rate | |
|---|---|---|
| | 2 min. | 15 min. |
| Example 4 | 5.4% | 14.4% |
| Example 5 | 12.8% | 41.3% |
| Comparative Example 2 | 63.9% | 87.5% |
| Comparative Example 3 | 51.6% | 71.5% |

(continued)

| Sample | Dissolution rate | |
|---|---|---|
| | 2 min. | 15 min. |
| Comparative Example 4 | 76.6% | 89.9% |
| Comparative Example 5 | 91.1% | 99.6% |

Experimental Example 4

Xanthan gum was added to a 50 mmol/L phosphate buffer solution at a concentration of 0.5 W/W% while stirring, and the acid addition salt of the present invention prepared in Example 1 (50 mg as the amount of mirabegron) was uniformly dispersed in the resulting xanthan gum solution while stirring, to prepare a suspension. The resulting suspension was orally administered to male beagle dogs using a sonde in a fasted state (Fasted) or after 30 minutes from the intake of a meal (Fed), and the drug concentration in the plasma was measured after predetermined periods of time. As the meal, 50 g of meat feed was used. Similarly, the drug aqueous solution prepared in Comparative Example 1 (50 mg as the amount of mirabegron) was orally administered to male beagle dogs using a sonde in a fasted state or after 30 minutes from the intake of a meal, and the drug concentration in the plasma was measured after predetermined periods of time.

The results of the acid addition salt of mirabegron of the present invention prepared in Example 1 are shown in Table 3 and Figure 3, and the results of Comparative Example 1 are shown in Table 4 and Figure 4. With respect to Comparative Example 1, the rate of decrease of Cmax in the fed state was 58%, and the rate of decrease of AUC was 43%, in comparison with those in the fasted state. With respect to the pharmaceutical composition of the present invention (Example 1), the rate of decrease of Cmax in the fed state was 22%, and the rate of decrease of AUC was 19%, in comparison with those in the fasted state. These results indicated that the change in pharmacokinetics caused by the presence or absence of food intake could be significantly reduced by the pharmaceutical composition of the present invention.

[Table 3]

| | Example 1 | |
|---|---|---|
| | Fasted | Fed |
| Cmax (ng/mL) | 295±166 | 229±86 |
| AUC0-10h (ng*hr/mL) | 1027±208 | 833±122 |

[Table 4]

| | Comparative Example 1 | |
|---|---|---|
| | Fasted | Fed |
| Cmax (ng/mL) | 439±58 | 183±75 |
| AUC0-10h (ng*hr/mL) | 1318±127 | 748±70 |

Experimental Example 5

Glass bottles were filled with a suspension prepared by dispersing the acid addition salt of the present invention prepared in Example 1 (25 mg as the amount of mirabegron) in 5 mL of a xanthan gum solution, and sealed. These bottles were allowed to stand in a cool place (5°C) or under room temperature conditions for a month. The xanthan gum solution was prepared by adding xanthan gum to water at a concentration of 1 W/W% while stirring. After the storage, each suspension (25 mg as the amount of mirabegron) was used to carry out a dissolution test in accordance with the dissolution test, method 2 (paddle method) described in the Japanese Pharmacopoeia. As the test solution, 900 mL of the second fluid (JP2) described in the disintegration test of the Japanese Pharmacopoeia was used. The paddle rotation speed was 150 rpm.

The results of the acid addition salt of the present invention prepared in Example 1 are shown in Figure 5. The change in dissolution rate at room temperature was 0.8% at the maximum, and no change in dissolution rate was

observed in the cool place. These results indicated that the leakage of the drug during the storage of the liquid could be inhibited.

Example 7

After 400 mg of an ethyl acrylate/methyl methacrylate/trimethylammoniumethyl methacrylate chloride copolymer (manufactured by EVONIK Roehm, product name: Eudragit RS100) was dissolved in methanol, the resulting solution was transferred to an agate mortar. To the agate mortar, 468 mg of the acid addition salt prepared in Example 1, and 64 mg of polyethylene glycol (manufactured by Sanyo Chemical Industries, Ltd., product name: PEG 6000; the same was used hereinafter) were further added, and mixed using a pestle. The agate mortar was transferred to an incubator, and dried at 40°C overnight. A film-like substance was peeled from the wall of the agate mortar, and pulverized using a pestle to obtain a pharmaceutical composition of the present invention.

Example 8

A mixture of 5.85 g of the acid addition salt prepared in Example 1 and 5 g of ethylcellulose (manufactured by Dow Chemical, product name: Ethocel STD 10; the same was used hereinafter) was granulated with 3.5 mL of methanol, using a compact agitation granulator (manufactured by ORIENTAL MOTOR Co., Ltd.; the same was used hereinafter). The resulting granules were dried at 40°C overnight, and pulverized to obtain a pharmaceutical composition of the present invention.

Example 9

A mixture of 5.85 g of the acid addition salt prepared in Example 1 and 15 g of ethylcellulose was granulated with 7.5 mL of methanol using a compact agitation granulator. The resulting granules were dried at 40°C overnight, and pulverized to obtain a pharmaceutical composition of the present invention.

Example 10

With respect to 8.775 g of the acid addition salt prepared in Example 1, 13.75 g of ethyl acrylate/methyl methacrylate/trimethylammoniumethyl methacrylate chloride copolymer (manufactured by EVONIK Roehm, product name: Eudragit RS30D) was added and granulated using a compact agitation granulator. The resulting granules were dried at 40°C overnight, and pulverized to obtain a pharmaceutical composition of the present invention.

Example 11

With respect to 8.775 g of the acid addition salt prepared in Example 1, 7.5 g of ethyl acrylate/methyl methacrylate/trimethylammoniumethyl methacrylate chloride copolymer (manufactured by EVONIK Roehm, product name: Eudragit RS30D) was added and granulated using a compact agitation granulator. The resulting granules were dried at 40°C overnight, and pulverized to obtain a pharmaceutical composition of the present invention.

Example 12

A spray liquid was prepared by adding 0.9 g of triethyl citrate (manufactured by Tokyo Chemical Industry Co., Ltd.; the same was used hereinafter) to 20 g of an aqueous dispersion of ethylcellulose (manufactured by FMC Corporation, product name: Aquacoat ECD-30; the same was used hereinafter) and irradiating the resulting mixture with a supersonic wave for 5 minutes. With respect to 5.85 g of the acid addition salt prepared in Example 1, 8.5 g of the spray liquid was added and granulated using a compact agitation granulator. The resulting granules were dried at 40°C overnight, and pulverized to obtain a pharmaceutical composition of the present invention.

Example 13

A spray liquid was prepared by adding 13.57 g of triethyl citrate to 300 g of an aqueous dispersion of ethylcellulose, irradiating the resulting mixture with a supersonic wave for 5 minutes, and passing it through a sieve of 250 μm. The acid addition salt prepared in Example 1 was pulverized using a power mill (manufactured by Dalton Corporation; the same was used hereinafter), and more finely pulverized using a fine impact mill (manufactured by Hosokawa Micron Corporation; the same was used hereinafter). With respect to 142.7 g of the pulverized acid addition salt, the total volume of the spray liquid prepared was added using an agitation granulator (manufactured by Powrex Corporation, product

name: VG-01), and the resulting granules were dried at 40°C overnight, and pulverized to obtain a pharmaceutical composition of the present invention.

Example 14

The acid addition salt prepared in Example 1 was pulverized using a power mill, and more finely pulverized using a fine impact mill. To 406.5 g of an aqueous dispersion of ethylcellulose, 71.3 g of the pulverized acid addition salt and 18.3 g of triethyl citrate were added, and the mixture was passed through a sieve of 250 μm to prepare a spray liquid. The resulting spray liquid was sprayed using a spray drier (manufactured by Ohkawara Kakohki Co., Ltd.; the same was used hereinafter). The particles were collected from the cyclone, and subjected to a heat treatment (70°C, 4 hours) to obtain a pharmaceutical composition of the present invention.

Example 15

The acid addition salt prepared in Example 1 was pulverized using a power mill, and more finely pulverized using a fine impact mill. To 813.0 g of an aqueous dispersion of ethylcellulose, 142.7 g of the pulverized acid addition salt and 36.6 g of triethyl citrate were added, and the mixture was passed through a sieve of 250 μm to prepare a spray liquid. The resulting spray liquid was sprayed using a spray drier. The particles were collected from the cyclone, and subjected to a heat treatment (70°C, 4 hours) to obtain a pharmaceutical composition of the present invention.

Comparative Example 6

To 406.8 of an aqueous dispersion of ethylcellulose, 125 g of mirabegron and 18.25 g of triethyl citrate were added, and the mixture was passed through a sieve of 250 μm to prepare a spray liquid. The resulting spray liquid was sprayed using a spray drier. The particles were collected from the cyclone, and subjected to a heat treatment (70°C, 4 hours) to obtain a pharmaceutical composition of a Comparative Example.

Comparative Example 7

To 543.48 of an aqueous dispersion of ethylcellulose, 250 g of mirabegron and 32.61 g of triethyl citrate were added, and 202.9 g of purified water was further added and mixed to prepare a spray liquid. The total volume of the resulting spray liquid was sprayed to 250 g of crystalline cellulose (manufactured by Asahi Kasei Chemicals Corporation, product name: CP-102Y; the same was used hereinafter) using a fluidized bed granulating apparatus (manufactured by Glatt, product name: GPCG-1; the same was used hereinafter), and subjected to a heat treatment (50°C, 13 hours) to obtain a pharmaceutical composition of a Comparative Example.

Comparative Example 8

(1) Preparation of first layer

A spray liquid was prepared by adding 240 g of mirabegron and 60 g of hypromellose (manufactured by Shin-Etsu Chemical Co., Ltd., product name: TC-5E) to 1200 g of purified water. The total volume of the resulting spray liquid was sprayed on 300 g of crystalline cellulose using a fluidized bed granulating apparatus to prepare particles coated with the first layer.

(2) Preparation of second layer

A spray liquid was prepared by adding 59 g of triethyl citrate to 983.26 g of an aqueous dispersion of ethylcellulose, and further adding thereto and mixing 983.26 g of purified water. The total volume of the resulting spray liquid was sprayed on 295 g of the particles coated with the first layer using a fluidized bed granulating apparatus, and subjected to a heat treatment (50°C, 13 hours) to obtain a pharmaceutical composition of a Comparative Example.

Experimental Example 6

The pharmaceutical compositions of the present invention prepared in Examples 7 to 15 (25 mg as the amount of mirabegron) were used to carry out a dissolution test in accordance with the dissolution test, method 2 (paddle method) described in the Japanese Pharmacopoeia. As the test solution, 900 mL of the second fluid (JP2) described in the disintegration test of the Japanese Pharmacopoeia was used. The paddle rotation speed was 150 rpm.

The results of the dissolution test of the pharmaceutical compositions of the present invention prepared in Examples 7 to 15 are shown in Figures 6 and 7. The dissolution rates after 30 minutes from the beginning of the test were 31% in Example 7, 26% in Example 8, 17% in Example 9, 24% in Example 10, 17% in Example 11, 32% in Example 12, 29% in Example 13, 36% in Example 14, and 29% in Example 15. The dissolution rate of mirabegron from each pharmaceutical composition prepared in Examples 7 to 15 was less than 85% after 30 minutes from the beginning of the test.

Experimental Example 7

The pharmaceutical composition of the present invention prepared in Example 7 (50 mg as the amount of mirabegron) was dispersed in a xanthan gum solution, and The pharmaceutical composition of the present invention prepared in Example 15 (50 mg as the amount of mirabegron) was dispersed in water. The resulting suspensions were orally administered to beagle dogs using a sonde in a fasted state or after 30 minutes from the intake of a meal, and the drug concentration in the plasma was measured. As the meal, 50 g of meat feed was used.

The results of pharmacokinetics of the pharmaceutical compositions of the present invention prepared in Examples 7 and 15 are shown in Table 5 and Figures 8 and 9.

With respect to the pharmaceutical compositions of the present invention (Examples 7 and 15), the rates of decrease of Cmax in the fed state were 4% and 8%, and the rates of decrease of AUC were -1% and 2%, in comparison with those in the fasted state. These results indicated that the change in pharmacokinetics caused by the presence or absence of food intake could be reduced by the pharmaceutical composition of the present invention, in comparison with the results of Comparative Example 1 (Table 4).

[Table 5]

| | Example 7 | | Example 15 | |
|---|---|---|---|---|
| | Fasted | Fed | Fasted | Fed |
| Cmax (ng/mL) | 204±42 | 201±49 | 209±73 | 193±57 |
| AUC0-10h (ng*hr/mL) | 706±78 | 711±22 | 880±239 | 860±320 |

Experimental Example 8

Glass bottles were respectively filled with a suspension prepared by dispersing the pharmaceutical composition of the present invention prepared in Example 7 in a xanthan gum solution, and another suspension prepared by dispersing the pharmaceutical composition of the present invention prepared in Example 15 in water, and sealed. These bottles were allowed to stand in a cool place (5°C) or under room temperature conditions for two weeks or a month. After the storage, each suspension (25 mg as the amount of mirabegron) was used to carry out a dissolution test in accordance with the dissolution test, method 2 (paddle method) described in the Japanese Pharmacopoeia. As the test solution, 900 mL of the second fluid (JP2) described in the disintegration test of the Japanese Pharmacopoeia was used. The paddle rotation speed was 150 rpm. Similarly, suspensions prepared by dispersing the pharmaceutical compositions prepared in Comparative Examples 6 to 8 in water were stored under the same conditions, and the dissolution test was carried out after the storage.

The results of the dissolution test of the pharmaceutical compositions of the present invention prepared in Examples 7 and 5 are shown in Figures 10 and 11, and the results of the dissolution test of the pharmaceutical compositions prepared in Comparative Examples 6 to 8 are shown in Figures 12 to 14. With respect to Comparative Examples 6 to 8, the changes in dissolution rate were 20% (room temperature), 16% (room temperature), and 55% (room temperature) at the maximum, respectively, in comparison with those before the storage. With respect to the Examples, the changes in dissolution rate were 0.8% (Example 7, cool place), 10% (Example 7, room temperature), and 5.3% (Example 15, room temperature), and these results indicated that the changes in dissolution were 15% or less, and that the leakage of the drug during the storage in each liquid could be inhibited.

Experimental Example 9

The pharmaceutical composition of the present invention prepared in Example 15 (150 mg as the amount of mirabegron) was respectively added to $10^{-1}$ mol/L hydrochloric acid, $10^{-3}$ mol/L hydrochloric acid, and purified water, and the mixtures were stirred. After one day, the mixtures were centrifuged at 3000 rpm for 15 minutes, and the drug concentration in each supernatant was measured. Similarly, the pharmaceutical compositions prepared in Comparative

Examples 7 and 8 were respectively added to $10^{-1}$ mol/L hydrochloric acid, $10^{-3}$ mol/L hydrochloric acid, and purified water, and the drug concentration in each supernatant was measured after one day from the addition, under the same conditions as those of Example 15.

The results of measuring drug concentrations in the pharmaceutical compositions prepared in Example 15 and Comparative Examples 7 and 8 are shown in Table 6. With respect to the pharmaceutical composition of the present invention prepared in Example 15, the solubility (drug concentration) could be inhibited in any solution. It is expected from these results that the bitterness can be inhibited.

[Table 6]

| Sample | Drug concentration in supernatant (mg/mL) | | |
|---|---|---|---|
| | $10^{-1}$ mol/L HCl | $10^{-3}$ mol/L HCl | Purified water |
| Example 15 | 0.006 | 0.001 | 0.004 |
| Comparative Example 7 | 0.007 | 0.555 | 0.461 |
| Comparative Example 8 | 0.120 | 0.816 | 0.837 |

Example 16

The acid addition salt prepared in Example 4 was pulverized using a power mill, and more finely pulverized using a fine impact mill. A dispersion was prepared from 504.4 g of an aqueous dispersion of ethylcellulose and 22.7 g of triethyl citrate. The total volume of the resulting dispersion was sprayed on 200 g of the pulverized acid addition salt using a fluidized bed granulating apparatus (inlet air temperature: 65°C, spray rate: 6 g/min). The resulting powder was collected and subjected to a heat treatment (70°C, 4 hours) to obtain a pharmaceutical composition of the present invention.

Example 17

The acid addition salt prepared in Example 6 was pulverized using a fine impact mill. A dispersion was prepared from 642.3 g of an aqueous dispersion of ethylcellulose and 28.9 g of triethyl citrate. The total volume of the resulting dispersion was sprayed to 175.8 g of the pulverized acid addition salt using a fluidized bed granulating apparatus (inlet air temperature: 65°C, spray rate: 6 g/min). The resulting powder was collected and subjected to a heat treatment (70°C, 4 hours) to obtain a pharmaceutical composition of the present invention.

Experimental Example 10

The pharmaceutical compositions of the present invention prepared in Examples 16 and 17 (25 mg as the amount of mirabegron) were used to carry out a dissolution test in accordance with the dissolution test, method 2 (paddle method) described in the Japanese Pharmacopoeia. As the test solution, 900 mL of a USP phosphate buffer (pH 6.8) was used. The paddle rotation speed was 200 rpm.

The results of the dissolution test of the pharmaceutical compositions of the present invention prepared in Examples 16 and 17 are shown in Figure 15. The dissolution rates of mirabegron after 30 minutes from the beginning of the test were 46% in Example 16 and 8% in Example 17. The dissolution rate of mirabegron from each pharmaceutical composition prepared in Examples 16 and 17 was less than 85% after 30 minutes from the beginning of the test.

Experimental Example 11

The pharmaceutical composition of the present invention prepared in Example 16 (25 mg as the amount of mirabegron) was dispersed in 12.5 mL of water. The resulting suspension was orally administered to beagle dogs using a sonde in a fasted state or after 30 minutes from the intake of a meal, and the drug concentration in the plasma was measured. As the meal, 50 g of meat feed was used.

The results of pharmacokinetics of the pharmaceutical composition of the present invention prepared in Example 16 are shown in Table 7 and Figure 16.

With respect to the pharmaceutical composition of the present invention (Example 16), the rate of decrease of Cmax in the fed state was -13%, and the rate of decrease of AUC was 18%, in comparison with those in the fasted state. From these results, a tendency to reduce the change in pharmacokinetics caused by the presence or absence of food intake, in comparison with the results of Comparative Example 1 (Table 4), was observed.

[Table 7]

| | Example 16 | |
|---|---|---|
| | Fasted | Fed |
| Cmax (ng/mL) | 47±8 | 53±30 |
| AUC0-10h (ng*hr/mL) | 281±13 | 231±54 |

Experimental Example 12

Glass bottles were respectively filled with suspensions prepared by dispersing the pharmaceutical compositions of the present invention prepared in Examples 16 and 17 in water, and sealed. These bottles were allowed to stand in a cool place (5°C) for two weeks. After the storage, each suspension (25 mg as the amount of mirabegron) was used to carry out a dissolution test in accordance with the dissolution test, method 2 (paddle method) described in the Japanese Pharmacopoeia. As the test solution, 900 mL of a USP phosphate buffer (pH 6.8) was used. The paddle rotation speed was 200 rpm.

The results of the dissolution test of the pharmaceutical compositions of the present invention prepared in Examples 16 and 17 are shown in Figures 17 and 18. The changes in dissolution rate at the maximum were 1.4% (5°C) in Example 16, and 2.3% (5°C) in Example 17, and these results indicated that the changes in dissolution were 15% or less, and that the leakage of the drug during the storage in each liquid could be inhibited.

Example 18

To a mixture of 600 g of mirabegron and 438 g of sodium lauryl sulfate, 4260 mL of water was added, and stirred and mixed at room temperature using a multipurpose mixer (manufactured by SHINAGAWA MACHINERY WORKS Co., Ltd., type 25AM-02-QR) at 65 rpm for 5 minutes. While following stirring and mixing, 1545.6 mL of 1 mol/L hydrochloric acid was added at 25 mL/min, and further stirred and mixed for 4 hours. The resulting mixture was tray-dried at 60°C for 15 hours to obtain an acid addition salt of mirabegron and lauryl sulfuric acid of the present invention at a molar ratio of 1:1. The resulting acid addition salt was pulverized using a fine impact mill. A dispersion of 800 g of an aqueous dispersion of ethylcellulose and 36 g of triethyl citrate was sprayed to 400 g of the pulverized acid addition salt using a fluidized bed granulating apparatus. The resulting particles were collected, passed through a sieve of 710 μm, and subjected to a heat treatment (70°C, 4 hours) to obtain a pharmaceutical composition of the present invention.

Example 19

To 576 g of the particles before the heat treatment, which was obtained in Example 18, a dispersion of 113.6 g of an aqueous dispersion of ethylcellulose and 5.1 g of triethyl citrate was further sprayed. The resulting particles were collected, passed through a sieve of 710 μm, and subjected to a heat treatment (70°C, 4 hours) to obtain a pharmaceutical composition of the present invention.

Example 20

To 515.2 g of the particles before the heat treatment, which was obtained in Example 19, a dispersion of 95.1 g of an aqueous dispersion of ethylcellulose and 4.3 g of triethyl citrate was further sprayed. The resulting particles were collected, passed through a sieve of 710 μm, and subjected to a heat treatment (70°C, 4 hours) to obtain a pharmaceutical composition of the present invention.

Example 21

To 400.1 g of the particles before the heat treatment, which was obtained in Example 20, a dispersion of 139 g of an aqueous dispersion of ethylcellulose and 6.3 g of triethyl citrate was sprayed using a fluidized bed granulating apparatus. The resulting particles were collected, passed through a sieve of 710 μm, and subjected to a heat treatment (70°C, 4 hours) to obtain a pharmaceutical composition of the present invention.

Example 22

To 348.1 g of the particles before the heat treatment, which was obtained in Example 21, a dispersion of 54 g of an aqueous dispersion of ethylcellulose and 2.4 g of triethyl citrate was further sprayed. The resulting particles were collected, passed through a sieve of 710 μm, and subjected to a heat treatment (70°C, 4 hours) to obtain a pharmaceutical composition of the present invention.

Example 23

To 266.7 g of the particles before the heat treatment, which was obtained in Example 22, a dispersion of 39.3 g of an aqueous dispersion of ethylcellulose and 1.8 g of triethyl citrate was further sprayed. The resulting particles were collected, passed through a sieve of 710 μm, and subjected to a heat treatment (70°C, 4 hours) to obtain a pharmaceutical composition of the present invention.

Experimental Example 13

The pharmaceutical compositions of the present invention prepared in Examples 20 and 23 (25 mg as the amount of mirabegron) were used to carry out a dissolution test in accordance with the dissolution test, method 2 (paddle method) described in the Japanese Pharmacopoeia. As the test solution, 900 mL of a USP phosphate buffer (pH 6.8) was used. The paddle rotation speed was 200 rpm.

The results of the dissolution test of the pharmaceutical compositions of the present invention prepared in Examples 20 and 23 are shown in Figure 19. The dissolution rates of mirabegron after 30 minutes from the beginning of the test were 34% in Example 20 and 15% in Example 23. The dissolution rate of mirabegron from each pharmaceutical composition prepared in Examples 20 and 23 was less than 85% after 30 minutes from the beginning of the test.

Experimental Example 14

The pharmaceutical composition of the present invention prepared in Example 23 (50 mg as the amount of mirabegron) was dispersed in 50 mL of water. The resulting suspension was orally administered to 6 beagle dogs using a sonde in a fasted state or after 30 minutes from the intake of a meal, and the drug concentration in the plasma was measured. As the meal, 50 g of meat feed was used.

The results of pharmacokinetics of the pharmaceutical composition of the present invention prepared in Example 23 are shown in Table 8 and Figure 20.

With respect to the pharmaceutical composition of the present invention (Example 23), the rate of decrease of Cmax in the fed state was -9%, and the rate of decrease of AUC was 15%, in comparison with those in the fasted state. From these results, a tendency to reduce the change in pharmacokinetics caused by the presence or absence of food intake, in comparison with the results of Comparative Example 1 (Table 4), was observed.

[Table 8]

| | Example 23 | |
|---|---|---|
| | Fasted | Fed |
| Cmax (ng/mL) | 188±37 | 204±48 |
| AUC0-10h (ng*hr/mL) | 929±85 | 787±132 |

Experimental Example 15

Glass bottles were respectively filled with suspensions prepared by dispersing the pharmaceutical compositions of the present invention prepared in Examples 20 and 23 in water, and sealed. These bottles were allowed to stand in a cool place (5°C) for two weeks. After the storage, each suspension (25 mg as the amount of mirabegron) was used to carry out a dissolution test in accordance with the dissolution test, method 2 (paddle method) described in the Japanese Pharmacopoeia. As the test solution, 900 mL of a USP phosphate buffer (pH 6.8) was used. The paddle rotation speed was 200 rpm.

The results of the dissolution test of the pharmaceutical compositions of the present invention prepared in Examples 20 and 23 are shown in Figures 21 and 22. The changes in dissolution rate at the maximum were 3.8% in Example 20, and 5.3% in Example 23, and these results indicated that the changes in dissolution were 15% or less,

and that the leakage of the drug during the storage of each liquid could be inhibited.

INDUSTRIAL APPLICABILITY

The pharmaceutical composition containing an acid addition salt of alkyl sulfuric acid and mirabegron, and a base for modified release can provide a pharmaceutical composition in which the dissolution or leakage of mirabegron can be reduced or inhibited when the pharmaceutical composition is suspended in a solvent, and in which the change in pharmacokinetics caused by the presence or absence of food intake is smaller, in comparison with a medicament containing mirabegron, and as a result, the pharmaceutical composition can improve the drug dosing compliance.

## Claims

1. A pharmaceutical composition comprising an acid addition salt of alkyl sulfuric acid and mirabegron, and a base for modified release.

2. The pharmaceutical composition according to claim 1, wherein the alkyl sulfuric acid is an acid selected from the group consisting of dodecyl sulfuric acid, tetradecyl sulfuric acid, and hexadecyl sulfuric acid.

3. The pharmaceutical composition according to claim 2, wherein the acid addition salt of alkyl sulfuric acid and mirabegron is mirabegron dodecyl sulfate.

4. The pharmaceutical composition according to any one of claims 1 to 3, wherein a molar ratio of mirabegron to alkyl sulfuric acid is 1:1 to 1:2.

5. The pharmaceutical composition according to claim 1, wherein the base for modified release is a water-soluble polymer or a water-insoluble substance.

6. The pharmaceutical composition according to claim 5, wherein the base for modified release is a water-insoluble substance.

7. The pharmaceutical composition according to claim 1 or 6, wherein the base for modified release is a water-insoluble cellulose ether and/or a water-insoluble acrylate copolymer.

8. The pharmaceutical composition according to claim 7, wherein the water-insoluble cellulose ether is ethyl cellulose.

9. The pharmaceutical composition according to claim 7, wherein the water-insoluble acrylate copolymer is one substance or two or more substances selected from an ethyl acrylate/methyl methacrylate/trimethylammoniumethyl methacrylate chloride copolymer and an ethyl acrylate/methyl methacrylate copolymer.

10. The pharmaceutical composition according to any one of claims 5 to 9, wherein a content of the water-insoluble substance is 0.1 W/W% or more and 1000 W/W% or less, with respect to the weight of the acid addition salt of alkyl sulfuric acid and mirabegron.

11. The pharmaceutical composition according to any one of claims 1 to 10, wherein a dissolution rate of mirabegron after 30 minutes from the beginning of a dissolution test is approximately less than 85%.

12. The pharmaceutical composition according to claim 11, wherein a dissolution rate of mirabegron after 1.5 hours from the beginning of a dissolution test is approximately 70% or less.

13. The pharmaceutical composition according to any one of claims 1 to 12, wherein a rate of decrease of a maximum blood drug concentration (Cmax) when administered after eating a meal, in comparison with a Cmax when administered in a fasted state, is approximately 30% or less.

14. The pharmaceutical composition according to any one of claims 1 to 13, wherein a rate of decrease of an area under a blood drug concentration versus time curve (AUC) when administered after eating a meal, in comparison with an AUC when administered in a fasted state, is approximately 30% or less.

**15.** The pharmaceutical composition according to any one of claims 1 to 14, wherein its dosage form is selected from the group consisting of granules, powders, liquids, suspensions, and emulsions.

**16.** The pharmaceutical composition according to claim 15, wherein the dosage form is selected from liquids, suspensions, and emulsions.

**17.** The pharmaceutical composition according to any one of claims 1 to 16, which is a therapeutic agent for overactive bladder.

**18.** A process of manufacturing a pharmaceutical composition, comprising adding a base for modified release to an acid addition salt of alkyl sulfuric acid and mirabegron.

**19.** A process of manufacturing a pharmaceutical composition, comprising the steps of:

(1) dissolving mirabegron in a solvent,
(2) adding alkyl sulfuric acid to the resulting mixture prepared in (1), and
(3) adding a base for modified release to the resulting mixture prepared in (2).

**20.** An acid addition salt of alkyl sulfuric acid and mirabegron.

**21.** The acid addition salt according to claim 20, wherein the alkyl sulfuric acid is an acid selected from the group consisting of dodecyl sulfuric acid, tetradecyl sulfuric acid, and hexadecyl sulfuric acid.

**22.** The acid addition salt according to claim 21, wherein the acid addition salt of alkyl sulfuric acid and mirabegron is mirabegron dodecyl sulfate.

**23.** The acid addition salt according to any one of claims 20 to 22, wherein a molar ratio of mirabegron to alkyl sulfuric acid is 1:1 to 1:2.

**24.** Use of the acid addition salt according to any one of claims 20 to 23 for the treatment of overactive bladder.

**25.** A method for treating overactive bladder, comprising administering to a subject in need thereof the acid addition salt according to any one of claims 20 to 23 in an amount effective therefor.

**26.** Use of the acid addition salt according to any one of claims 20 to 23 for the inhibition of leakage of mirabegron during the storage of mirabegron dispersed in water or a xanthan gum solution.

**27.** Use of the acid addition salt according to any one of claims 20 to 23 for the manufacture of a pharmaceutical composition in which the change in pharmacokinetics is reduced regardless of the presence or absence of food intake.

**28.** Use of the acid addition salt according to any one of claims 20 to 23 for the inhibition of bitterness.

**29.** Use of the acid addition salt according to any one of claims 20 to 23 for the manufacture of a pharmaceutical composition for the treatment of overactive bladder.

Figure 1

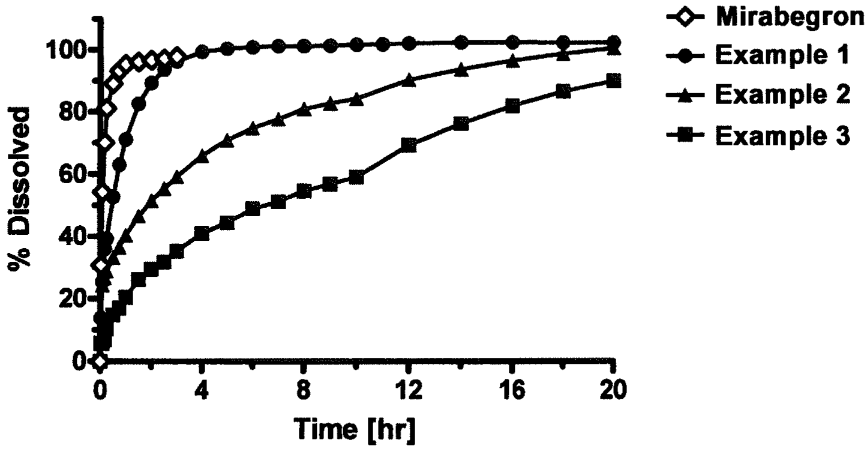


Figure 2

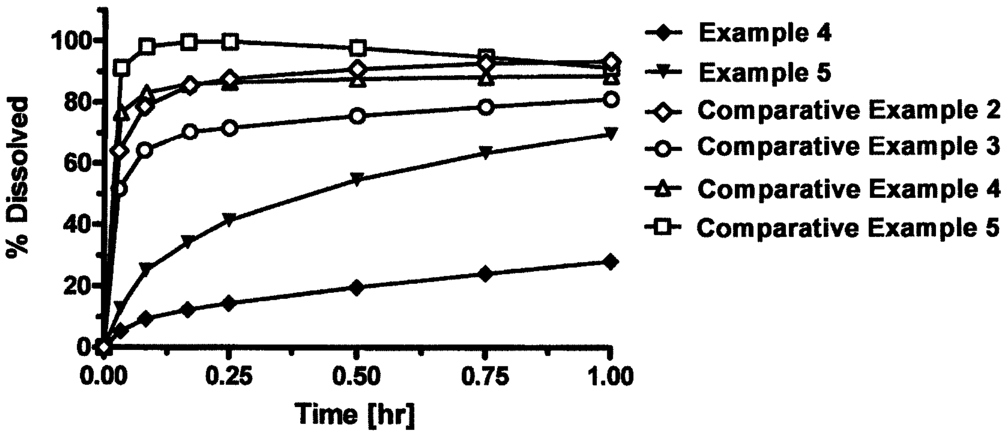


Figure 3

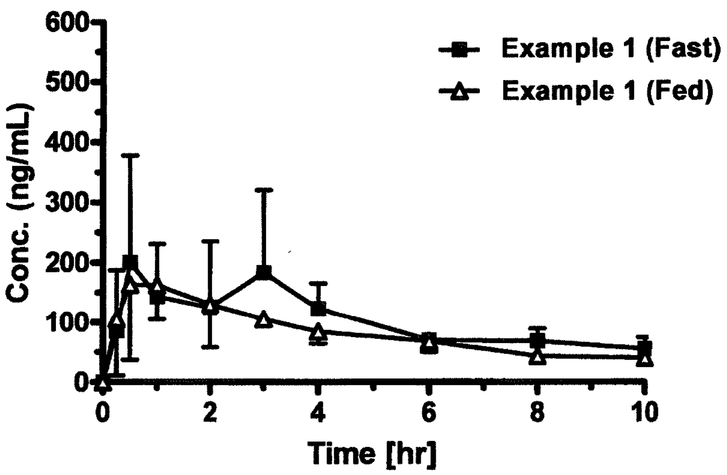

Figure 4

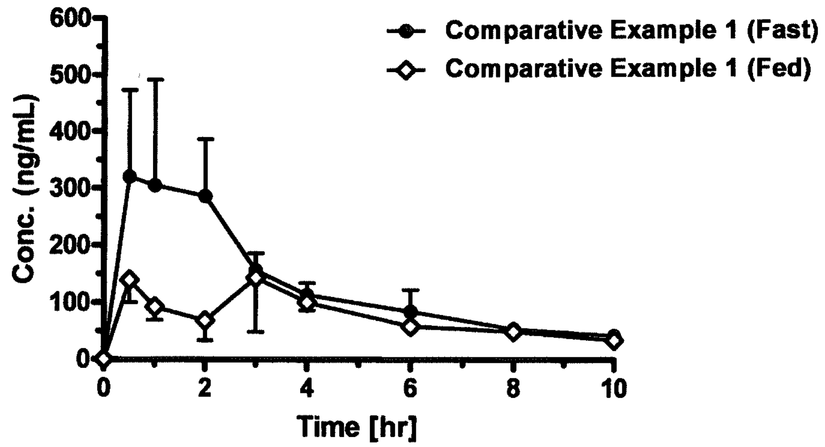


Figure 5

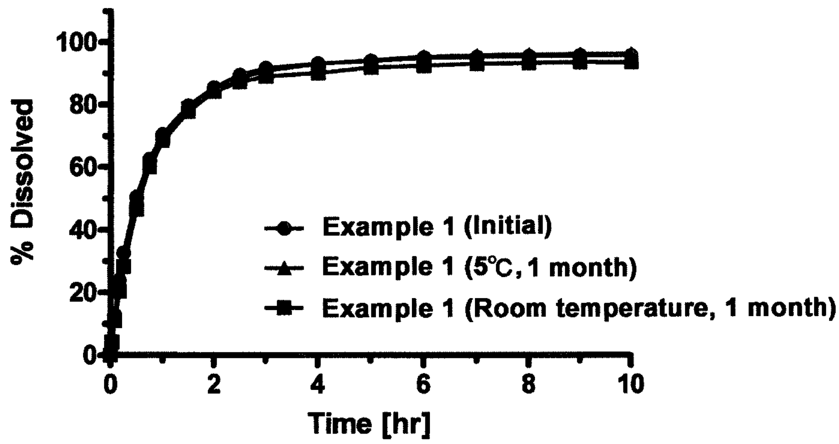


Figure 6

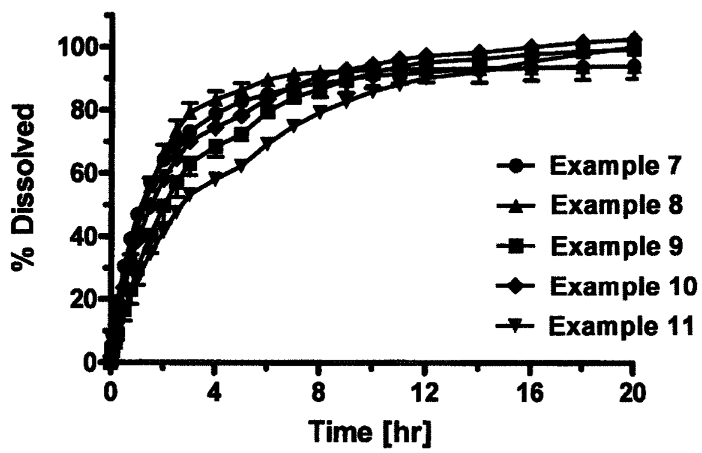

Figure 7

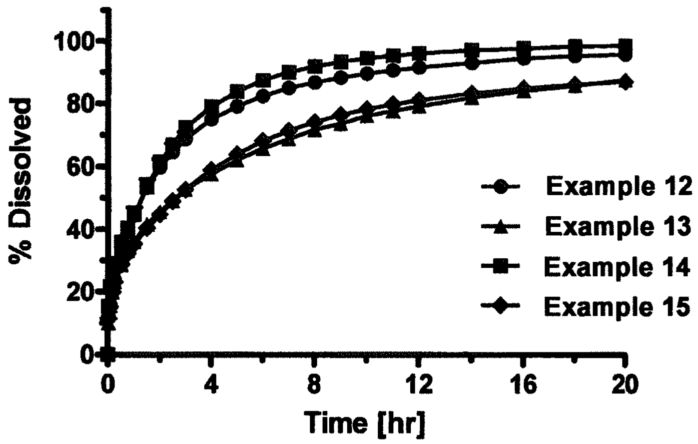


Figure 8

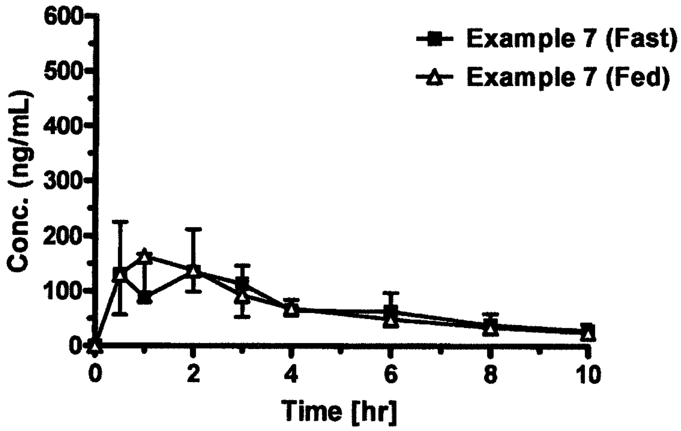


Figure 9

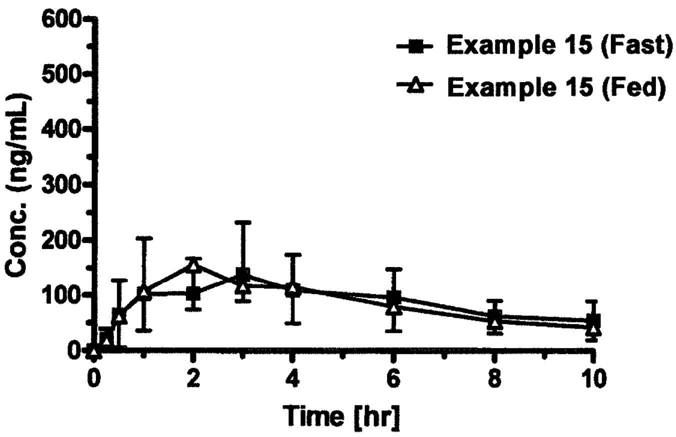

Figure 10

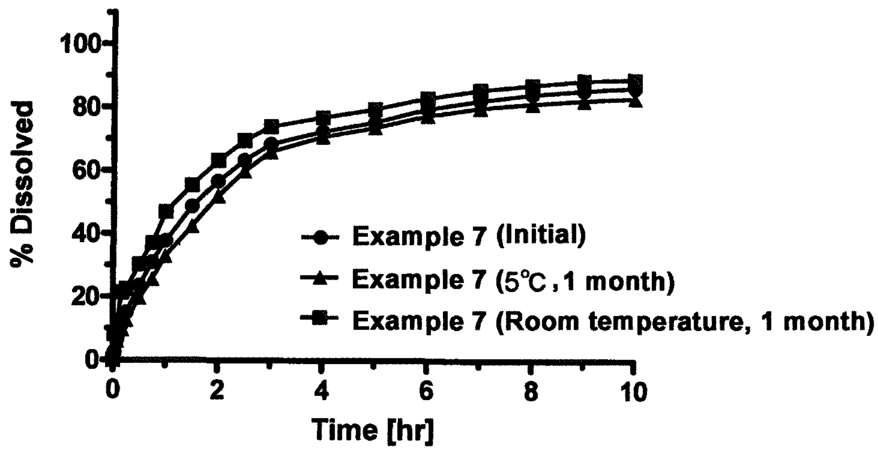


Figure 11

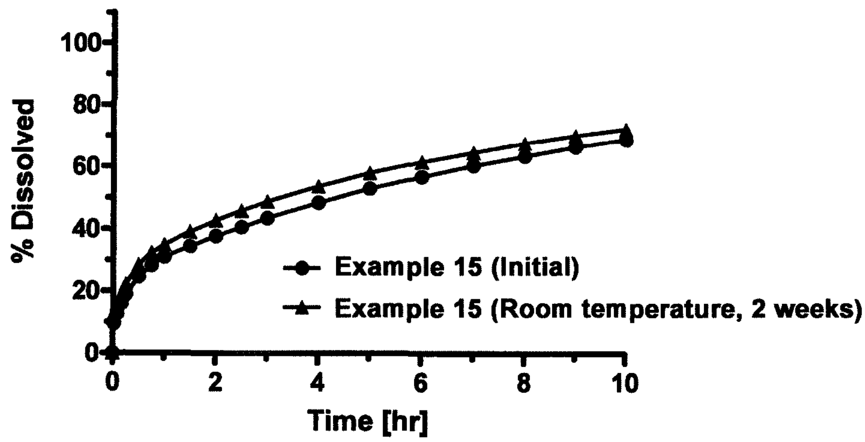


Figure 12

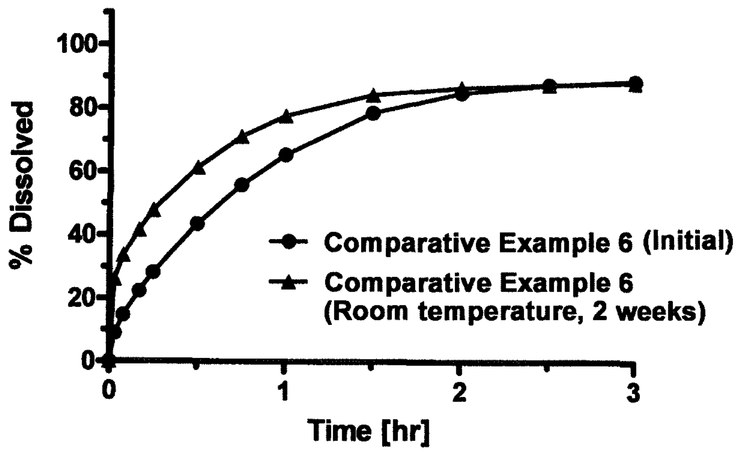

Figure 13

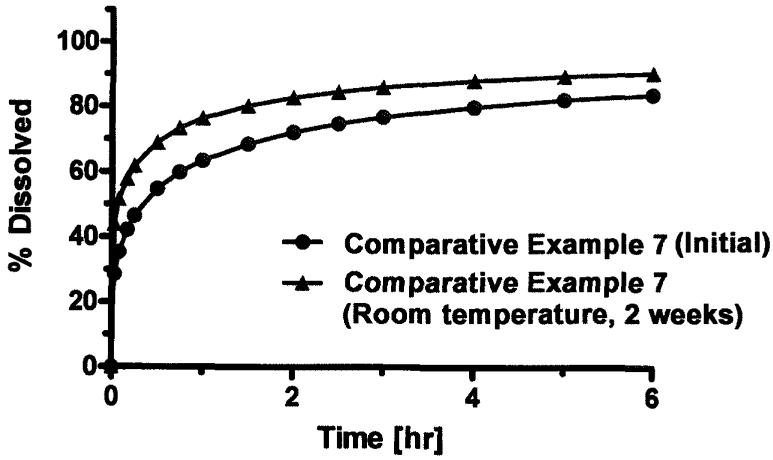


Figure 14

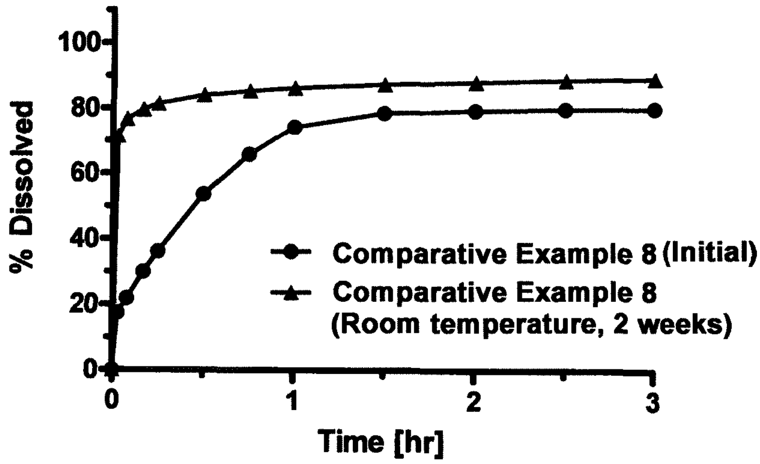


Figure 15

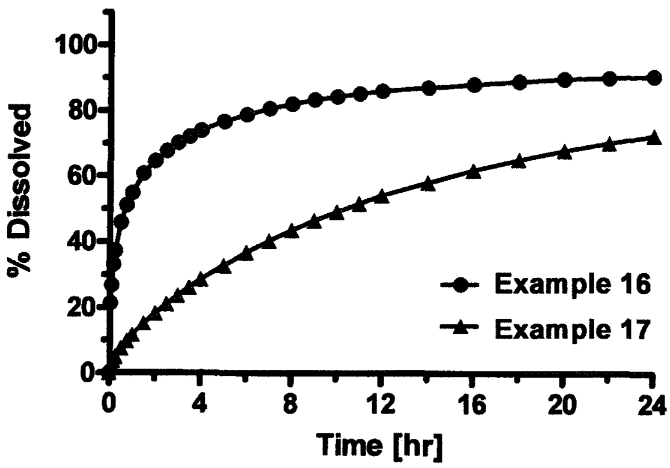

Figure 16

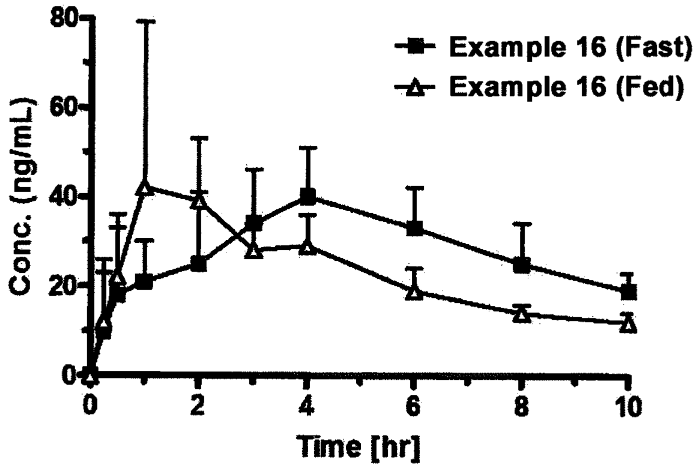


Figure 17

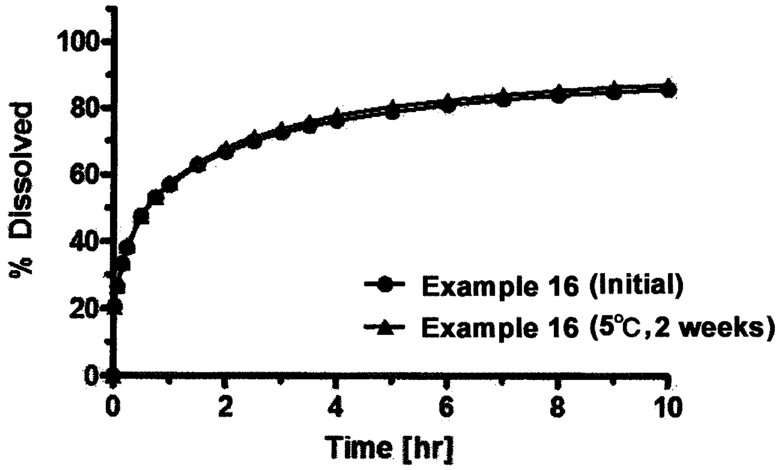


Figure 18

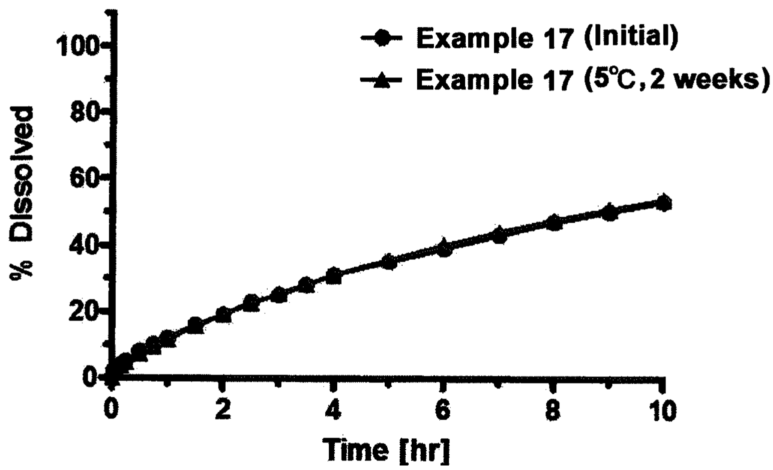

Figure 19

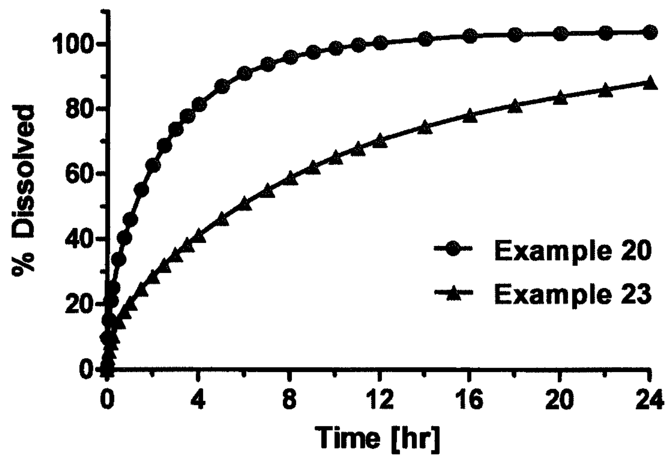


Figure 20

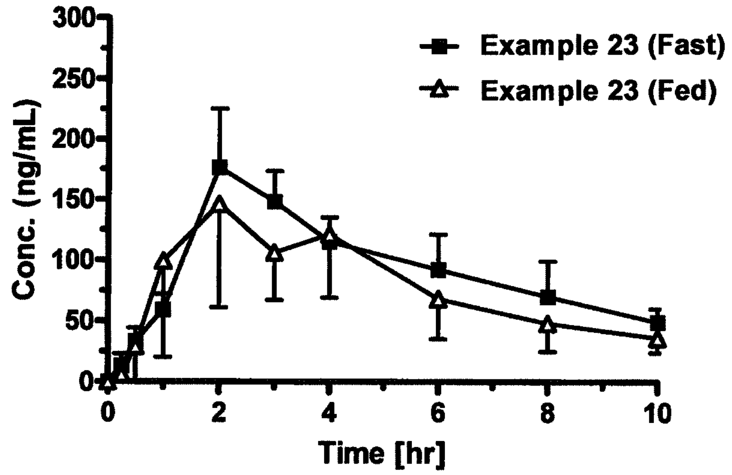


Figure 21

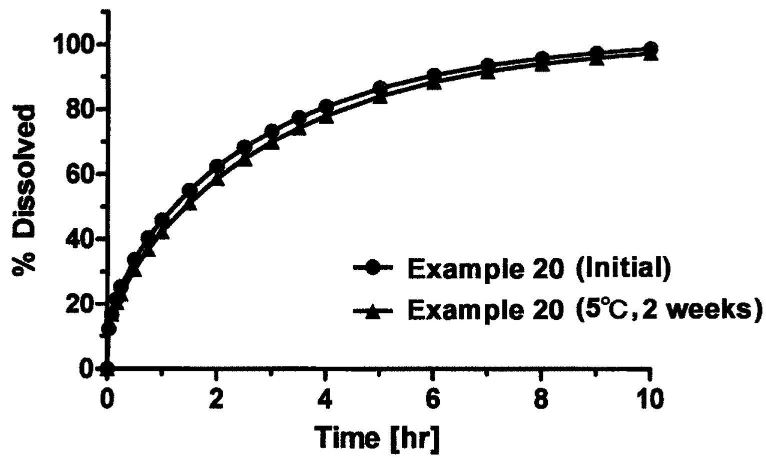

Figure 22

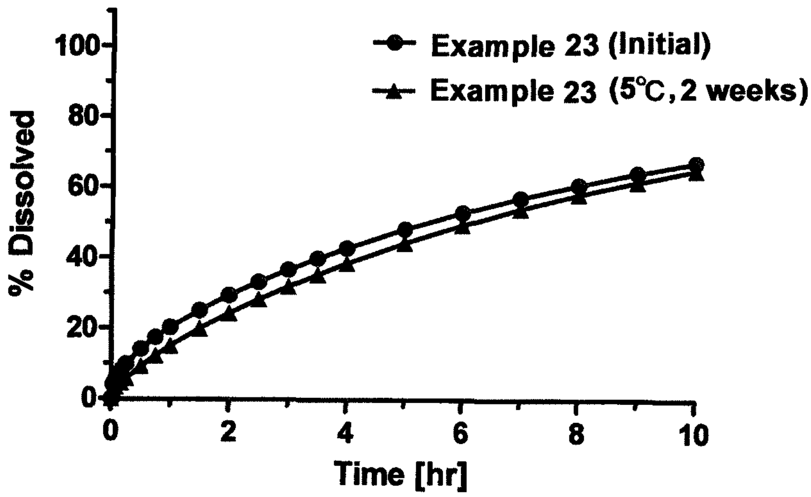
Example 23 (Initial)
Example 23 (5℃, 2 weeks)
% Dissolved
100
80
60
40
20
0
0
2
4
6
8
10
Time [hr]

## INTERNATIONAL SEARCH REPORT

International application No.
PCT/JP2013/059486

A. CLASSIFICATION OF SUBJECT MATTER
*C07D277/20*(2006.01)i, *A61K31/426*(2006.01)i, *A61K47/32*(2006.01)i, *A61K47/38*(2006.01)i, *A61P13/10*(2006.01)i

According to International Patent Classification (IPC) or to both national classification and IPC

B. FIELDS SEARCHED

Minimum documentation searched (classification system followed by classification symbols)
C07D277/20, A61K31/426, A61K47/32, A61K47/38, A61P13/10

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CAplus/REGISTRY(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| Y | WO 99/20607 A1 (Yamanouchi Pharmaceutical Co., Ltd.), 29 April 1999 (29.04.1999), entire text; particularly, example 41; page 5, lines 9 to 13; page 14, lines 3 to 6 & US 6346532 B1 & EP 1028111 A1 | 1-23,26-29 |
| Y | WO 2010/053068 A1 (Dainippon Sumitomo Pharma Co., Ltd.), 14 May 2010 (14.05.2010), entire text; particularly, test example 3 & US 2011/0262566 A1 & EP 2363397 A1 | 1-23,26-29 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 May, 2013 (10.05.13) | 21 May, 2013 (21.05.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2013/059486

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2010/038690 A1 (Astellas Pharma Inc.), 08 April 2010 (08.04.2010), entire text; particularly, paragraphs [0002], [0007] & JP 2011-79859 A & US 2010/0144807 A1 & EP 2345410 A1 | 1-23,26-29 |
| Y | WO 99/033491 A1 (Yamanouchi Pharmaceutical Co., Ltd.), 08 July 1999 (08.07.1999), entire text; particularly, claims; page 3, line 13 to page 5, line 4; page 9, line 15 to page 10, line 4; experimental example 2 & JP 2006-111636 A & US 6328979 B1 & US 6919372 B1 & EP 1043031 A1 & EP 1043030 A1 & WO 1999/033489 A1 & WO 1999/033490 A1 | 1-23,26-29 |
| Y | WO 99/033489 A1 (Yamanouchi Pharmaceutical Co., Ltd.), 08 July 1999 (08.07.1999), entire text; particularly, claims; page 3, line 13 to page 5, line 4; page 12, lines 8 to 24; experimental example 3 & JP 2006-111636 A & US 6328979 B1 & US 6919372 B1 & EP 1043030 A1 & EP 1043031 A1 & WO 1999/033490 A1 & WO 1999/033491 A1 | 1-23,26-29 |
| Y | JP 05-279247 A (Rohto Pharmaceutical Co., Ltd.), 26 October 1993 (26.10.1993), entire text; particularly, claims; paragraph [0001] & US 5368852 A & EP 565301 A1 | 1-19 |
| Y | JP 05-213740 A (Dainippon Pharmaceutical Co., Ltd.), 24 August 1993 (24.08.1993), entire text; particularly, claims; paragraph [0044] (Family: none) | 1-19 |

Form PCT/ISA/210 (continuation of second sheet) (July 2009)

**INTERNATIONAL SEARCH REPORT**

International application No.
PCT/JP2013/059486

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 24,25
because they relate to subject matter not required to be searched by this Authority, namely:
Claims 24 and 25 pertain to methods for treatment of the human body by surgery or therapy and thus relate to a subject matter on which this International Searching Authority is not required to carry out a search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004041276 A
- WO 9920607 A
- WO 03037881 A
- WO 2010038690 A
- WO 9933489 A
- WO 9933491 A


### Non-patent literature cited in the description


- *ICH Guidance: ICH, Guidance for Industry: E11 Clinical Investigation of Medical Products in the Pediatric Population,* December 2000
- *FDA: Guidance for Industry: General Consideration for Pediatric Pharmacokinetic Studies for Drugs and Biological Products,* November 1998
- *PREA: Guidance for Industry: How to Comply with the Pediatric Research Equity Act,* September 2005